# EUROPEAN PATENT APPLICATION

(11) **EP 4 417 201 A1**
(43) Date of publication of application: **21.08.2024**
(21) Application number: 23157270.2
(22) Date of filing: 17.02.2023
(51) Int. Cl.: A61K 31/404, A61K 31/122, A61K 45/06, A61P 35/00

(54) **PHARMACEUTICAL COMPOSITIONS FOR USE IN CANCER TREATMENT**

(71) Applicant: Robert Bosch Gesellschaft für medizinische Forschung mbH, 70376 Stuttgart (DE)
(72) Inventor: The designation of the inventor has not yet been filed
(74) Representative: Ullrich & Naumann PartG mbB

(57) **Abstract**

The present invention relates to pharmaceutical compositions comprising chalcone compounds and in particular to pharmaceutical compositions comprising chalcone compounds of *Formula A* for use in the treatment of cancer. Embodiments of the invention have been particularly developed for use in the treatment of malignant pleural mesothelioma (MPM), renal cell carcinoma (RCC) or diffuse large B-cell lymphomas (DLBCL) and will be described hereinafter with reference to this application. The invention also relates to further uses of the chalcone compounds of *Formula A.*

## Description

### FIELD OF THE INVENTION

The present invention relates to pharmaceutical compositions comprising chalcone compounds and in particular to pharmaceutical compositions comprising chalcone compounds of *Formula A* for use in the treatment of cancer. Embodiments of the invention have been particularly developed for use in the treatment of malignant pleural mesothelioma (MPM), renal cell carcinoma (RCC) or diffuse large B-cell lymphomas (DLBCL) and will be described hereinafter with reference to this application. The invention also relates to further uses of the chalcone compounds of *Formula A.*

### BACKGROUND OF THE INVENTION

Any discussion of the background art throughout the specification should in no way be considered as an admission that such art is widely known or forms part of common general knowledge in the field

Malignant pleural mesothelioma (MPM), renal cell carcinoma (RCC) or diffuse large B-cell lymphomas (DLBCL) are malignant tumor diseases with poor prognosis and often severely limited treatment options. In the fight against these tumors, in addition to surgical removal of the tumor tissue, treatment with drugs, i.e. chemotherapy or combined chemo-immunotherapy, is carried out as a complementary therapeutic measure or - in the case of non-operable tumors - even as the sole therapeutic measure. However, unfortunately the currently used therapeutic agents i) are only effective in some patients (often only in the minority of patients); and ii) have a nonspecific effect, i.e. they damage both tumor cells and healthy body cells, and are, therefore, often associated with serious side effects. Such that an urgent need for improved treatments exists.

In the context of MPM, the best possible therapies complete surgical resection of the pleura in combination with a cisplatin/pemetrexed- or a cisplatin/raltitrexed-chemotherapy. Due to the regularly multifocal and diffuse phenotype of MPM, surgical resection is only an option for a subset of patients. Furthermore, the majority of MPM tumors is resistant to chemotherapy: even with the currently recommended chemotherapy regiments, response rates of only 25 to 35% and a mean survival time of 12 months are achieved. Currently, for cases where this treatment regimen fails, no recommendation for an alternative or second choice treatment regimen exists.

Inhibition of aberrantly activated molecules by specific inhibitors has shown remarkable clinical response in various cancers and may *per se* be a promising strategy to cure MPM. Inhibition of key molecules in MPM pathogenesis, mTOR or kinases as EGFR, VEGFR, MET, did suppress MPM tumor cell growth in pre-clinical models but was not effective in clinical trials. In combination therapy, there is a therapeutic benefit in adding the VEGFR inhibitor bevacizumab to cisplatin/pemetrexed. An alternative strategy using immune checkpoint inhibitors (anti-PD-1, anti-PD-L1 antibodies) alone or in combination therapy showed promising therapeutic efficacy, achieving objective response in 19-29% of patients. Nonetheless, there is still an unmet need for therapeutic agents that efficiently target cell division and induce cell death in MPM cells.

RCC is one of the top ten cancers and the only curative option available is surgical resection. Furthermore, most RCC tumors are resistant to chemotherapy and various treatment attempts using immunotherapy, for example administering interferon of tumor vaccines have been unsuccessful. In the last couple of years, various combination therapies using kinase inhibitors and immune checkpoint inhibitors have become available but the tumors regularly develop resistance to these agents such that about 40% of patients with a primary localized RCC relapse. As a consequence, the 5-year survival rate of patients with metastasizing RCC is still below 20%.

DLBCL is the most frequent subtype of malignant Non-Hodgkin Lymphoma and the majority of deaths caused by Non-Hodgkin Lymphomas are attributed to DLBCL. While treatment with a combination of chemotherapeutic and immunotherapeutic agents comprising rituximab, cyclophosphamide, doxorubicin hydrochloride, vincristine and prednisolone (R-CHOP) can achieve stable remission in about 60 to 70% of DLBCL patients, about one third of all patients relapse within 6 to 12 months following treatment due to resistances against the therapeutic agents. Any remaining therapeutic options require the patient to be in a very good overall condition, are highly expensive and are typically associated with severe side effects due to toxicity.

There is a need in the art for improved therapeutic agents, which predominantly target MPM, RCC and DLBCL tumor cells and which have only minor effects on healthy cells such that the currently-prevalent side effects of chemotherapeutic treatment can be reduced and, at best, largely avoided.

It is an object of the present invention to overcome or ameliorate at least one of the disadvantages of the prior art, or to provide a useful alternative. In particular, it is an object of the present invention to provide improved pharmaceutical compositions for use in the treatment of malignant pleural mesothelioma (MPM), renal cell carcinoma (RCC) or diffuse large B-cell lymphomas (DLBCL).

### SUMMARY OF THE INVENTION

Malignant pleural mesothelioma (MPM), renal cell carcinoma (RCC) or diffuse large B-cell lymphomas (DLBCL) are malignant neoplasms with dismal prognosis and notorious chemotherapeutic resistance. Therefore, chemotherapeutic compounds and pharmaceutical compositions comprising the same that are able to specifically target MPM, RCC and/or DLBCL cells but have only minor effects on healthy cells are urgently needed.

The inventors have surprisingly found that a compound of ***Formula A*:** 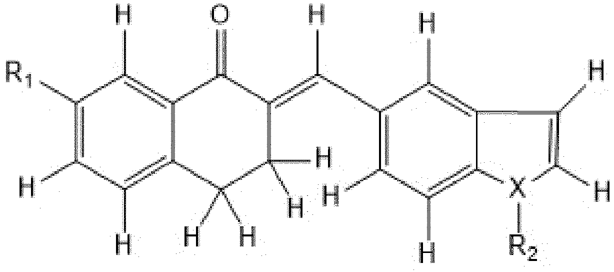 wherein:
X is a heteroatom;
R₁ is halo; and
R₂ is a C₁-C₁₀ alkyl,
induces MPM, RCC and/or DLBCL cell death. In particular, the inventors found that the MPM, RCC and/or DLBCL cell toxicity of the compound of Formula A is independent of Stathmin (STMN1) and/or collapsin response mediator protein-2 (CRMP2) and that the compound therefore may be used as a therapeutic agent for treating tumors irrespective of their STMN1 and CRMP2 expression.

Accordingly, in a first aspect, the present invention relates to a pharmaceutical composition comprising a compound of ***Formula A*** 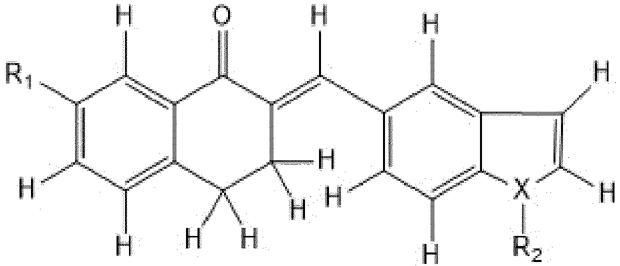 wherein:
X is a heteroatom;
R₁ is halo; and
R₂ is a C₁-C₁₀ alkyl,
for use in the treatment of malignant pleural mesothelioma (MPM), renal cell carcinoma (RCC) or diffuse large B-cell lymphomas (DLBCL).

Preferably, the treatment is treatment of MPM, RCC or DLBCL partially or fully resistant to other chemotherapeutic agents and/or immunotherapeutic agents.

In some embodiments, the treatment is treatment of MPM, RCC or DLBCL comprising cells negative for stathmin (STMN1) and/or collapsin response mediator protein-2 (CRMP2).

Typically, the compound of Formula A is a compound of ***Formula A-1*** 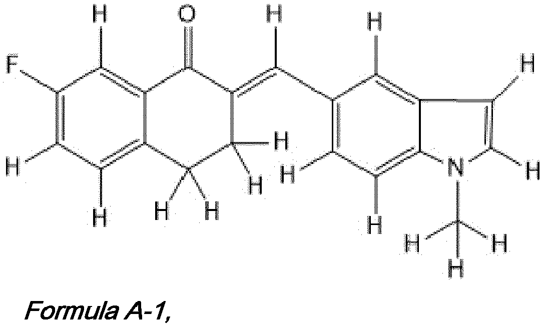 or a compound of ***Formula A-2***

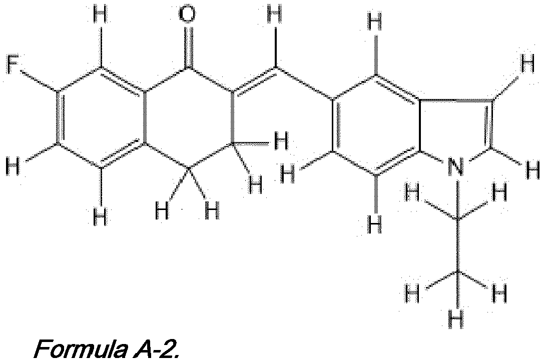

In a second aspect, the present invention relates to use of a compound of ***Formula A*** 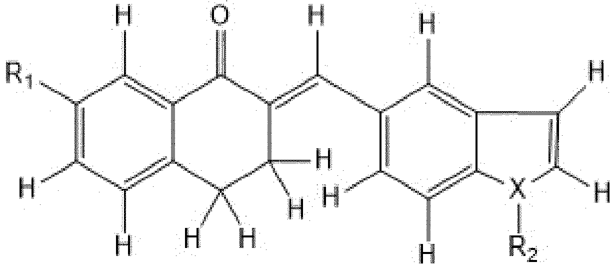 wherein:
X is a heteroatom;
R₁ is halo; and
R₂ is a C₁-C₁₀ alkyl,
for disturbing the polymerization of tubulin molecules.

In a third aspect, the present invention relates to use of a compound of ***Formula A*** 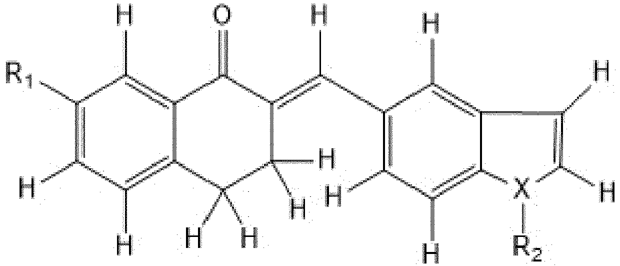 wherein:
X is a heteroatom;
R₁ is halo; and
R₂ is a C₁-C₁₀ alkyl,
for inhibiting formation of a functional spindle apparatus.

In both the second and third aspects, the use of a compound of ***Formula A*** is use of a compound of ***Formula A-1*** 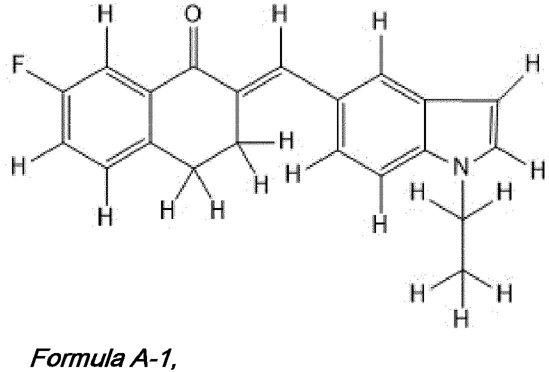 or a compound of ***Formula A-2***

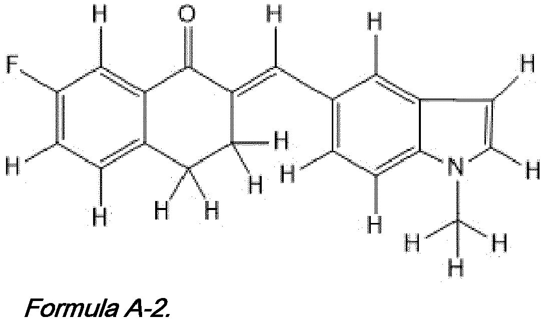

In some embodiments of the second and third aspect, the use of a compound of Formula A, preferably of *Formula A-1* or *A-2,* is in cells, such as in MPM cells, RCC cells or DLBCL cells, which are negative for stathmin (STMN1) and/or collapsin response mediator protein-2 (CRMP2).

### FIGURES

Embodiments of the invention will now be described, by way of example only, with reference to the accompanying drawings in which:
- **Fig. 1**: in accordance with the experiments described in Example 1, illustrates the effects of compounds of Formula A on the viability of MPM-PDX cell lines NCI-H2052 **(A),** NCI-H28 **(B)** and MSTO-211H **(C).** Lines, mean ± SD of independent triplicate experiments.

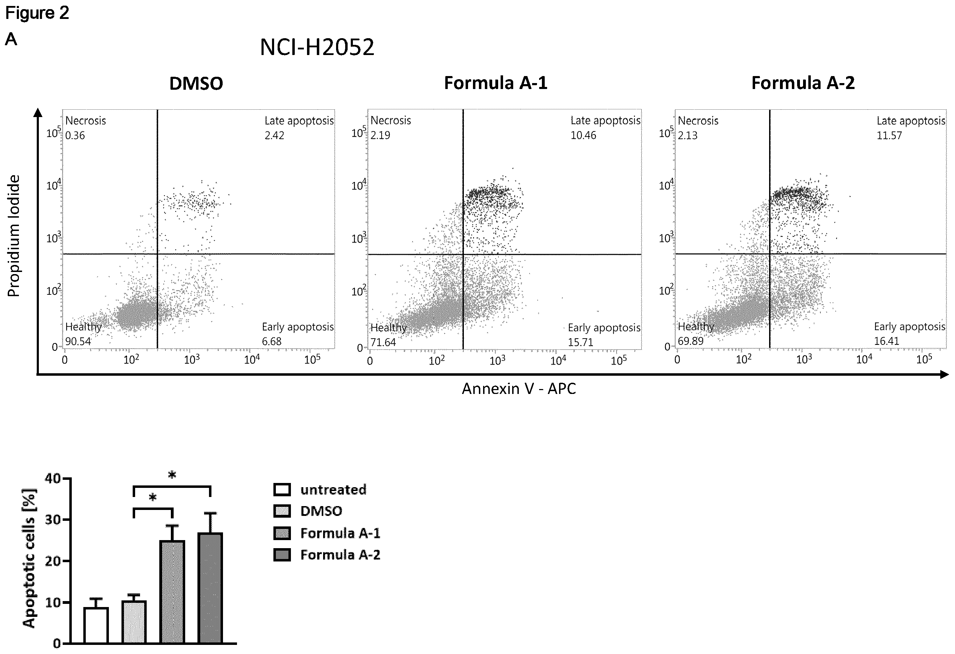

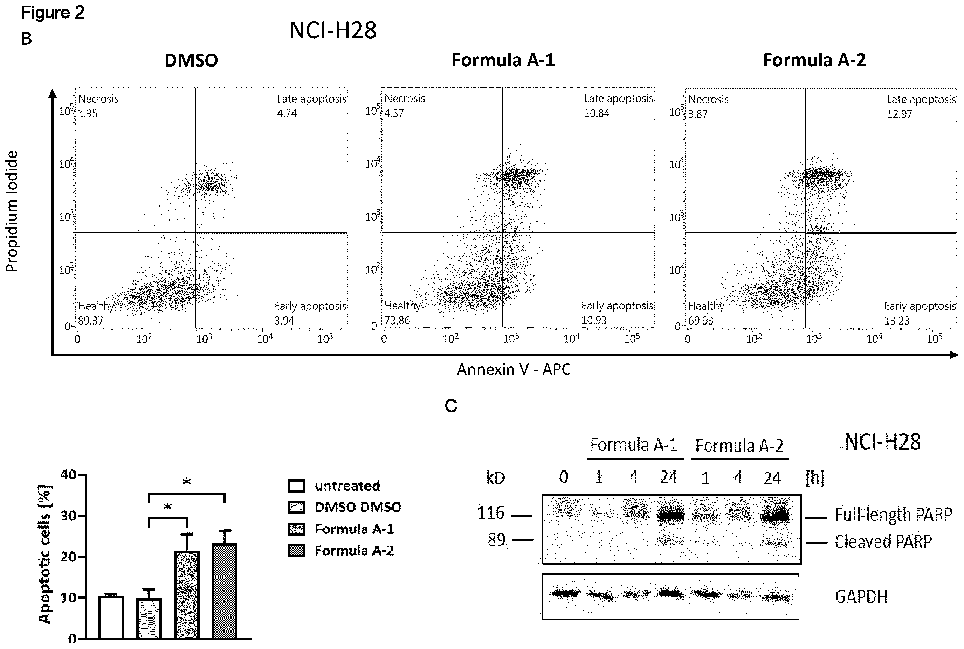

- **Fig. 2**: in accordance with the experiments described in Examples 1 and 2, illustrates the effects of compounds of *Formula A-1* (500 nM) and *A-2* (500 nM) on apoptosis induction in MPM cells detected by flow cytometry using Annexin-V/PI staining, compared to DMSO (0.005%). MPM cell line NCI-H2052 **(A)** and MPM cell line NCI-H28 (B). PhosphadidykAnnexin V binding of apoptotic cells and propidium iodide (PI) staining of late apoptotic and necrotic cells were recorded after 24h. Data shown as mean of two independent experiments (one-way Anova statistics with Tukey's multiple comparisons test; *, p<0.05). (C) PARP cleavage (intrinsic apoptotic marker) at 24 h treatment with *Formula A-1* (1 µM) and *Formula A-2* (1 µM) detected by immunoblotting in NCI-H28 cells.
- **Fig. 3**: in accordance with the experiments described in Examples 1 and 2, illustrates apoptotic membrane blebbing of NCI-H28 and NCI-H2052 cells exposed to DMSO (0.003%) **(A),** *Formula A-1* (300 nM) **(B)** and *Formula A-2* (300 nM) (C). Scale, 50 µm.
- **Fig. 4**: in accordance with the experiments described in Example 1, illustrates the effects of compounds of *Formula A-1* **(A)** and *Formula A-2* **(B)** on the viability of MPM, RCC and DLBCL cells compared to their effects on fibroblasts.
- **Fig. 5**: in accordance with the experiments described in Example 1, illustrates the effects of compounds of *Formula A-1* **(A)** and *Formula A-2* **(B)** on the viability of RCC cell lines 7860 and A498. Lines, mean ± SD of independent triplicate experiments.
- **Fig. 6**: in accordance with the experiments described in Example 1, illustrates the effects of compounds of *Formula A-1* **(A)** and *Formula A-2* **(B)** on the viability of DLBCL cell lines U2932 and OciLy19. Lines, mean ± SD of independent triplicate experiments.

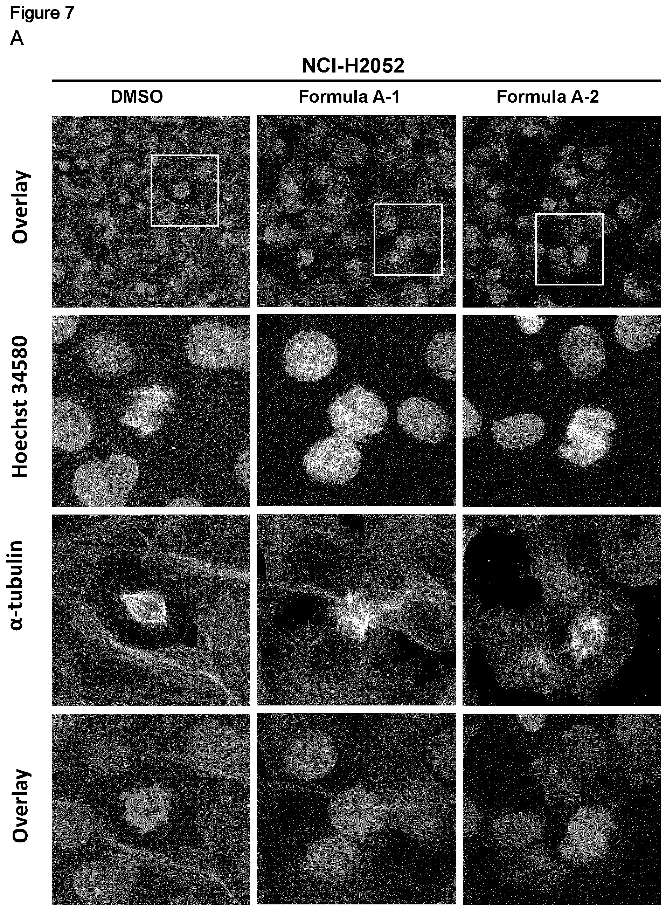

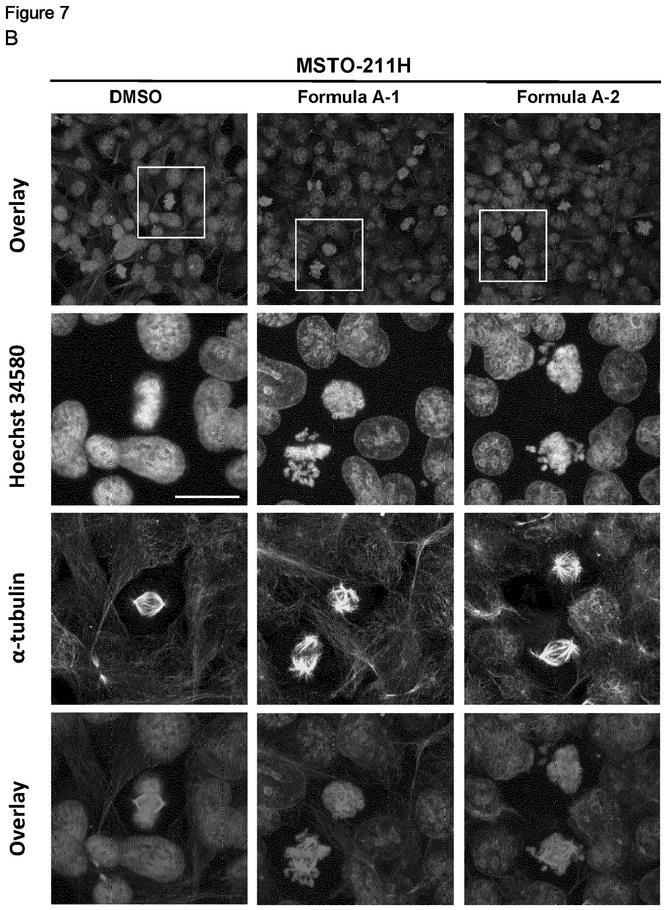

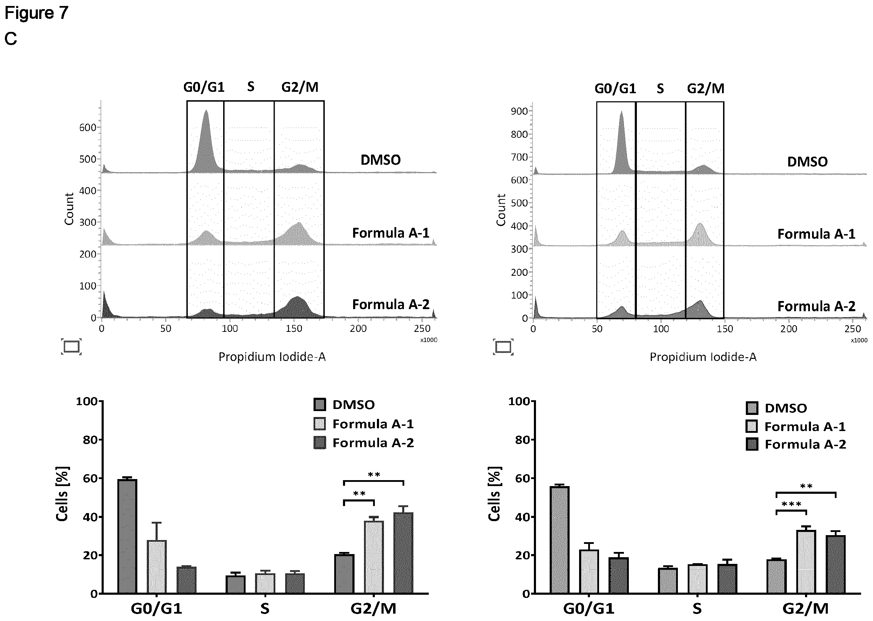

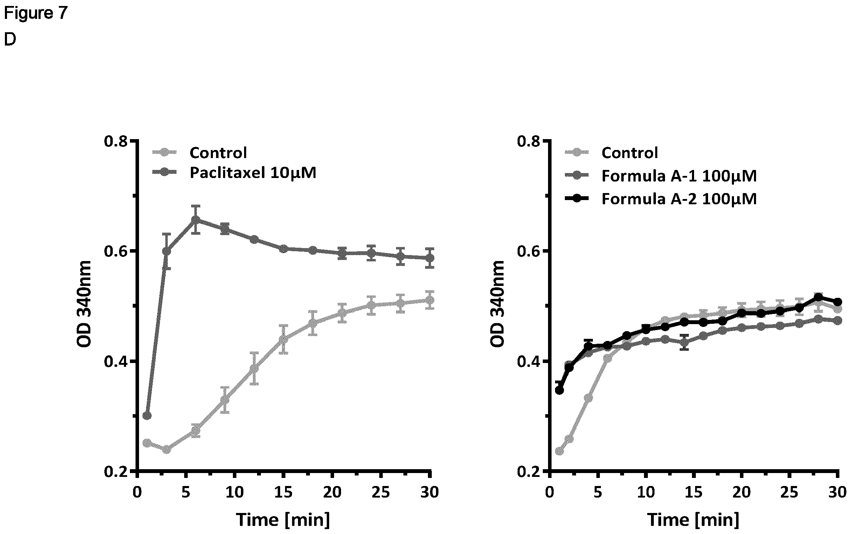

- **Fig. 7**: in accordance with the experiments described in Example 3, illustrates the effects of compounds of *Formula A* on the formation of tubulin fibers in MPM cells. NCI-H2052 **(A)** and NCI-H28 cells **(B)** were stained using α-tubulin antibody (red) and Hoechst34580 (blue) and analyzed by confocal laser scanning microscopy after treatment with 100 nM *Formula A-1* or *Formula A-2* or 0.001 % DMSO for 24 h. Scale bar, 20 µm. Representative experiments are shown. (C) Cell cycle analysis by flow cytometry using PI-staining after treatment of NCI-H2052 and NCI-H28 cells with *Formula A*-*1* (100 nM) or *Formula A-2* (100 nM) for 14 h, compared to DMSO (0.001%). Data shown as mean of three independent experiments (One-Way ANOVA; **P<0.005, ***P=0.0003). (D) Purified tubulin was incubated with *Formula A-1* (100 µM), *Formula A-2* (100 µM), paclitaxel (10 µM) or buffer alone. Microtubule polymerization was measured kinetically at 340 nm at 35°C. Data shown as mean ± SD of two replicate samples for each treatment, representative of two independent experiments.
- **Fig.8**: in accordance with the experiments described in Example 4, illustrates the role of STMN1 and CRMP2 in mediating the anti-tumor effects of compounds of *Formula A*-*1* and *A-2* on the viability of MPM, RCC and DLBCL cell lines.
**(A)** All cell lines expressed moderate (NCI-H28, A489) or high levels of STMN1 (NCI-H2052, MSTO-211H, 7860, U2932, Ocily19), whereas
**(B)** significant amounts of CRMP2 were only expressed in NCI-H28 and MSTO-211H, both determined by immunohistochemistry. Scale bar, 100 µm.
**(C)** Immunoblotting with STMN1 and CRMP2 specific antibodies of NCI-H28 and MSTO-211H cells treated with 1 µM *Formula A-1* or 1 µM *Formula A-2* for 1 h, 4 h and 24 h. NCI-H2052 **(D)** and MSTO-211H **(E)** cells were transfected with STMN1-siRNA or non-target control (NTC)-siRNA. Temporary knockdown of STMN1 was confirmed by immunoblotting. Cell viability of transfected NCI-H2052 **(D)** and MSTO-211H **(E)** cells exposed to the indicated concentrations of *Formula A*-*1* and *Formula A-2* for 72h was measured by MTT assay. Mean ± SD of four replicate samples. Statistical significance was determined by Two-Way ANOVA, ****P<0.0001; *P<0.05, ns=P>0.05.

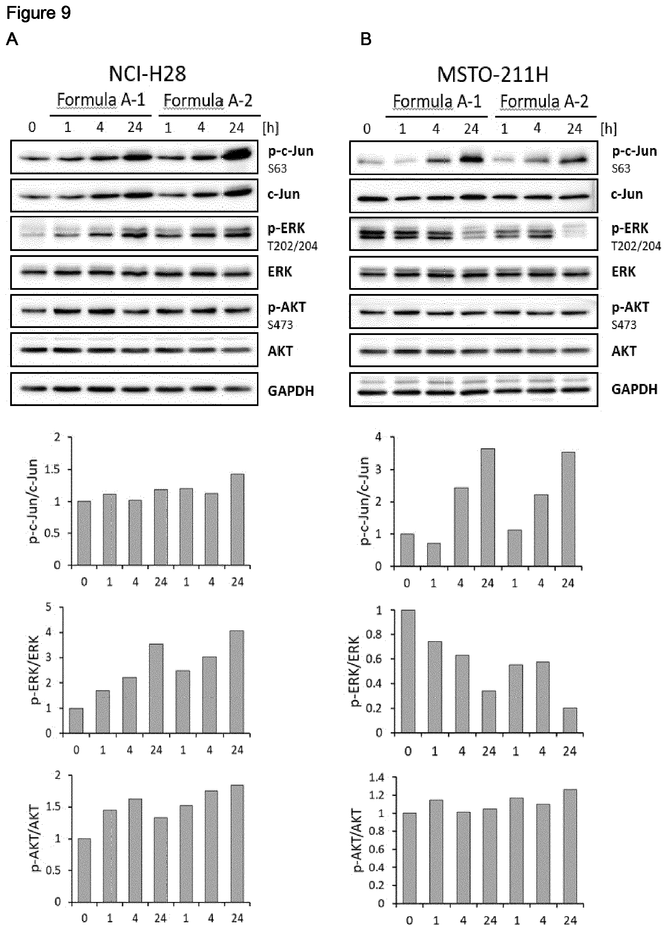

- **Fig. 9**: in accordance with the experiments described in Example 5, illustrates the effects of compounds of *Formula A* on intracellular signaling in MPM cells. Immunoblot analysis of signaling molecules in NCI-H28 **(A)** and MSTO-211H **(B)** cells treated with 1 µM *Formula A-1* or 1 µM *Formula A-2* for 1 h, 4 h and 24 h. Bar graphs represent relative p-S63/total c-Jun, p-T202/204/total ERK and p-S473/total AKT protein levels in chalcone compound and untreated cells. Data are representative of two independent experiments.

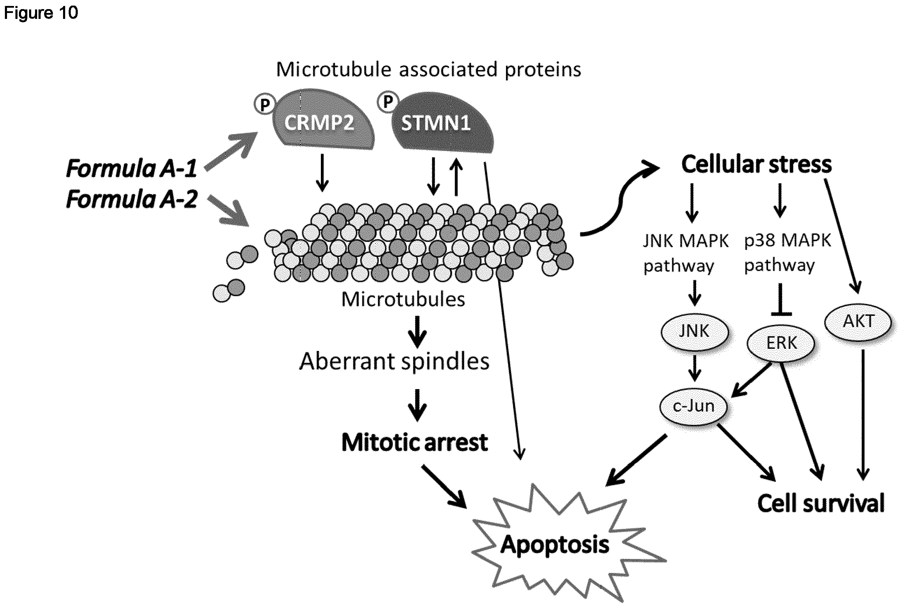

- **Fig. 10**: illustrates the molecular action of compounds of *Formula A.* Compounds of *Formula A* promote the assembly of aberrant microtubule fibers via direct interaction with tubulin and phosphorylation of microtubule regulatory proteins STMN1 and CRMP2. Formation of aberrant tubulin fibers induces abnormal spindle morphology, mitotic arrest and apoptosis. Apoptosis is mediated by activation of pro-apoptotic signaling (STMN1, JNK/c-Jun) and inhibition of ERK, thus counteracting stress-induced pro-survival signaling (c-Jun, AKT).

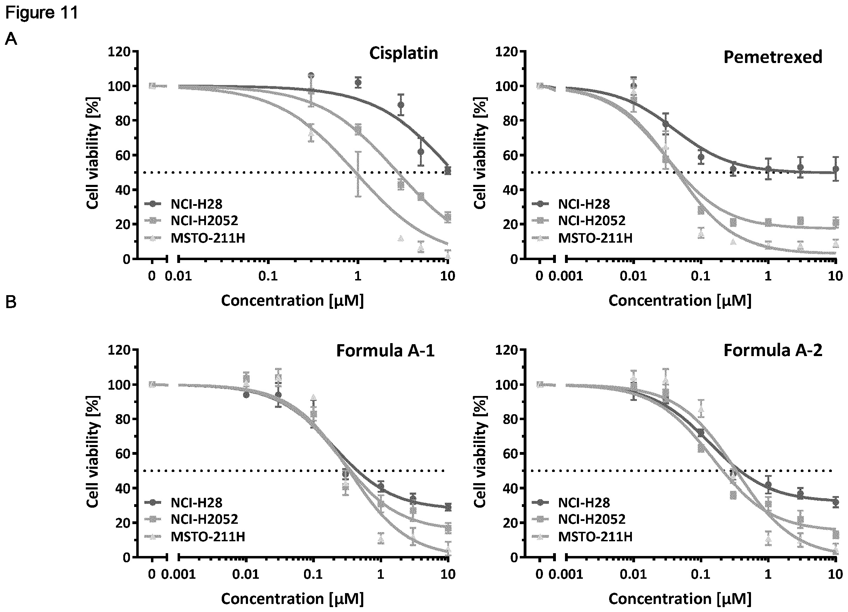

- **Fig. 11**: in accordance with the experiments described in Example 6, illustrates a comparative assessment of the effects of **(A)** standard MPM therapeutics cisplatin and pemetrexed as well as of **(B)** compounds of *Formula A*-*1* and *A-2* on MPM cell lines NCI-H2052, NCI-H28 and MSTO-H211.

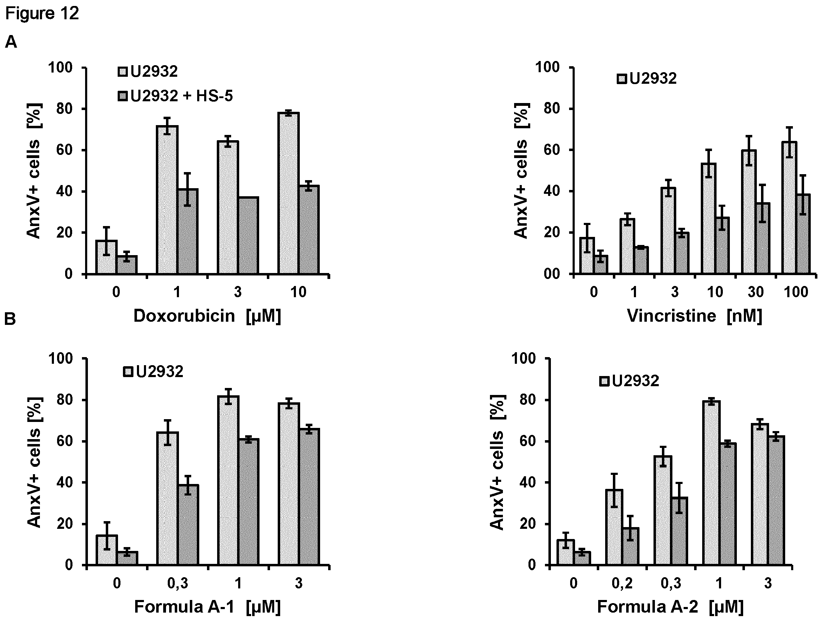

- **Fig. 12**: in accordance with the experiments described in Example 7, illustrates a comparative assessment of the effects of **(A)** standard DLBCL therapeutics doxorubicin and vincristine as well as of **(B)** compounds of *Formula A*-*1* and *A-2* on DLBCL cells U2932 either cultured in the presence or absence of HS-5 stromal cells.

### DETAILED DESCRIPTION OF THE INVENTION

In order to provide a clear and consistent understanding of the specification and claims, and the scope to be given such terms, the following definitions are provided.

### Definitions

In the context of the present disclosure, the term "negative for stathmin (STMN1) and/or collapsin response mediator protein-2 (CRMP2)" means that cells do not express STMN1 protein or CRMP2 protein or neither of them at significant levels. STMN1 and CRMP2 expression is assessed by estimating the intensity of immunohistochemistry staining: 0/no staining; 1+/faint staining; 2+/moderate; 3+/strong staining in at least 10% cells. Cells showing no or faint staining are regarded as negative, cells with moderate or strong staining are regarded as positive for the respective protein. The term "negative for stathmin (STMN1)" after knockdown of STMN1 protein expression by siRNA means that cells expressed less than 20% of the original amount of STMN1, verified by immunoblotting.

The term "heteroatom" refers to an atom selected from the group consisting of nitrogen (N), oxygen (O) and sulphur (S).

The term "alkyl" refers to a saturated, linear or branched hydrocarbon moiety, such as -CH₃ or -CH(CH₃)₂.

In the context of the present disclosure, the term "chemotherapeutic agent" refers to a molecule such as a chemical compound, which has cytotoxic or at least cytostatic properties towards cells of malignant neoplasms and can, thereby, stop or at least slow the growth of a malignant tumor. Examples of chemotherapeutic agents include doxorubicin hydrochloride, vincristine, everolimus, cisplatin, cyclophosphamide, pemetrexed, raltitrexed, temsirolimus, sunitinib, and axitinib.

In the context of the present disclosure, the term "immunotherapeutic agent" refers to a polypeptide molecule that specifically recognizes and binds a target sequence or epitope of an antigen and, thereby, mediates tumor cell death. In the context of the present specification, the term particularly refers to polypeptide molecules that recognize and bind to a specific epitope of a "cancer antigen", i.e. of an antigen that shows tumor-specific expression, thereby inhibiting the activity of the aberrant cancer antigen or to mark the respective cell for better recognition by the endogenous immune response. Accordingly, an immunotherapeutic agent in the context of the present specification essentially relies on the immunoglobulin (Ig) concept of target/epitope recognition known from molecules of the conventional Ig format (i.e. IgG, IgD, IgE, IgA and/or IgM) and, in particular, includes monoclonal antibodies directed against cancer antigens, such as rituximab, avelumab, ipilimumab, nivolumab and bevacizumab.

However, immunotherapeutic agents can also be of another format such as in the format of an antibody fragment or derivative, bi- or tri-specific antibody constructs, diabodies, camelid antibodies, domain antibodies, nanobodies or chimeric antigen receptors (CARs).

In the context of the present disclosure, the term "partially or fully resistant to other chemotherapeutic agents and/or immunotherapeutic agents" means that a tumor does not respond to treatment (fully resistant tumor) or that a tumor does respond only partially, leading to decreased tumor growth but not to tumor shrinkage (partially resistant tumor). The cancer may be resistant at the beginning of treatment, or it may become resistant during treatment leading to tumor relapse. In the course of *in vitro* therapy studies, cell lines that do not respond to treatment (75-100% surviving cells at high concentrations of the agent) are resistant to the therapeutic agent. Cell lines that do respond partially (45-75% surviving cells at high concentrations of the agent) are regarded partially resistant.

In addition to the above definitions, and unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

Further, reference throughout this specification to "one embodiment", "some embodiments" or "an embodiment" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

As used herein, the term "exemplary" is used in the sense of providing examples, as opposed to indicating quality. That is, an "exemplary embodiment" is an embodiment provided as an example, as opposed to necessarily being an embodiment of exemplary quality.

Inhibition of aberrantly activated molecules by specific inhibitors has shown remarkable clinical response in various cancers and may *per se* be a promising strategy to cure MPM, RCC and/or DLBCL.

Inhibition of key molecules in MPM pathogenesis, mTOR or kinases as EGFR, VEGFR, MET, did suppress MPM tumor cell growth in pre-clinical models but was not effective in clinical trials. In combination therapy, there is a therapeutic benefit in adding the VEGFR inhibitor bevacizumab to cisplatin/pemetrexed. An alternative strategy using immune checkpoint inhibitors (anti-PD-1, anti-PD-L1 antibodies) alone or in combination therapy showed promising therapeutic efficacy, achieving objective response in 19-29% of patients.

As indicated above, RCC is one of the top ten cancers and the most common type of kidney cancer in adults, responsible for approximately 90-95% of kidney cancer cases. Initial treatment is most commonly either partial or complete surgical removal of the affected kidney(s). However, inoperable tumors regularly develop resistance against the standard chemotherapeutic agents. While various combination therapies using kinase inhibitors and immune checkpoint inhibitors have become available in recent years, about 40% of patients with a primary localized RCC relapse due to resistance. As a consequence, the 5-year survival rate of patients with metastasizing RCC is still below 20%.

DLBCL biology is heterogeneous which is reflected by the marked variability in the clinical outcome. Chemotherapy regimens (CHOP) combined with anti-CD20 antibodies (Rituximab) represent the standard of care in patients with symptomatic disease. Although a high proportion of patients achieve complete remission, therapy resistance or relapse occurs in 30-40% DLBCL patients. The clinical management of patients with DLBCL is complicated by a rapid evolution of drug resistance primarily due to the ability of malignant B cells to interact with tumor-supportive microenvironment. In particular, stromal cells sustain the survival, proliferation, and drug resistance of malignant B cells.

In light of the above, it is clear that there is still an unmet need for therapeutic agents that efficiently target tumor cell division and induce cell death in MPM, RCC and DLBCL cells.

Chalcone derivatives are novel, highly efficacious anticancer agents with minimal toxicity. These compounds bear a chalcone scaffold, two rings with delocalized electrons linked through a ketovinyl chain, which is very reactive and enables the addition of reactive groups and pharmacophores of known therapeutic potential. Depending on their substituents, the anticancer mechanisms of chalcone derivatives are induction of apoptosis, microtubule binding and cell cycle arrest, or enzyme inhibition.

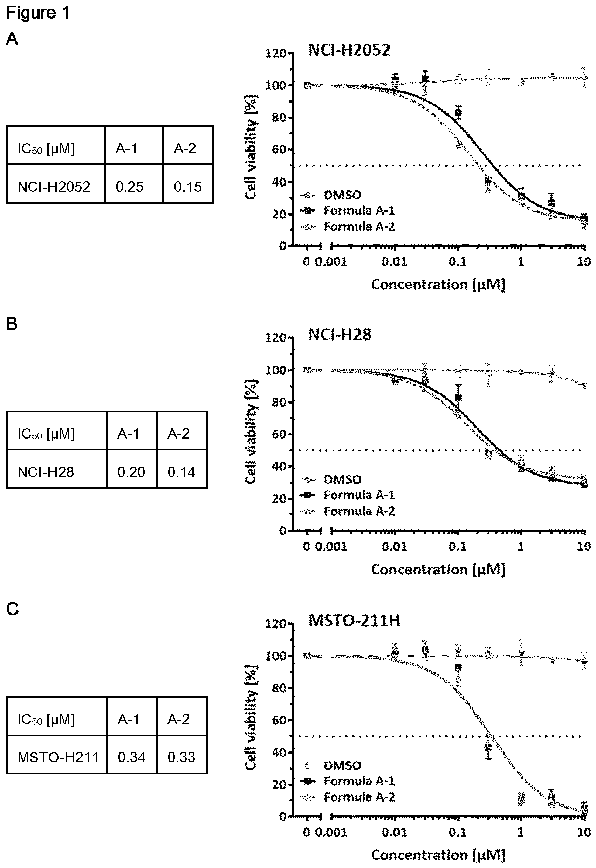

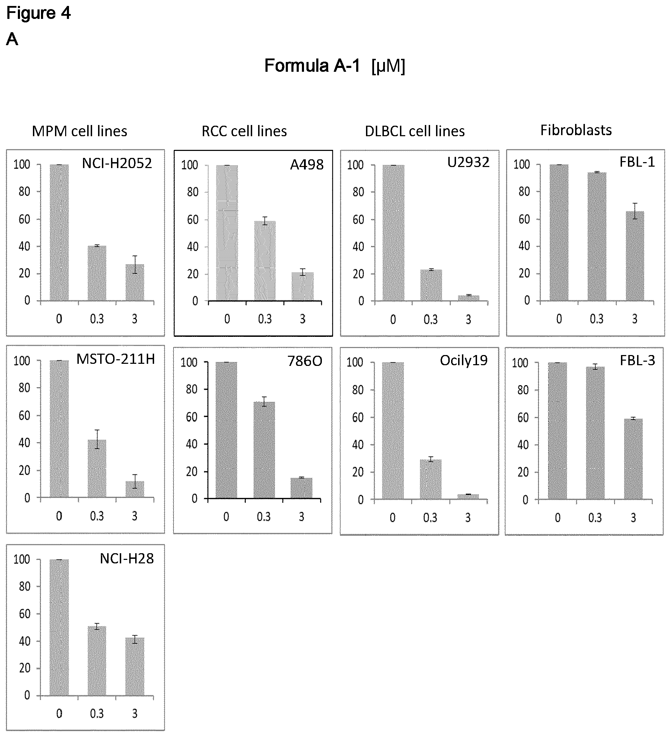

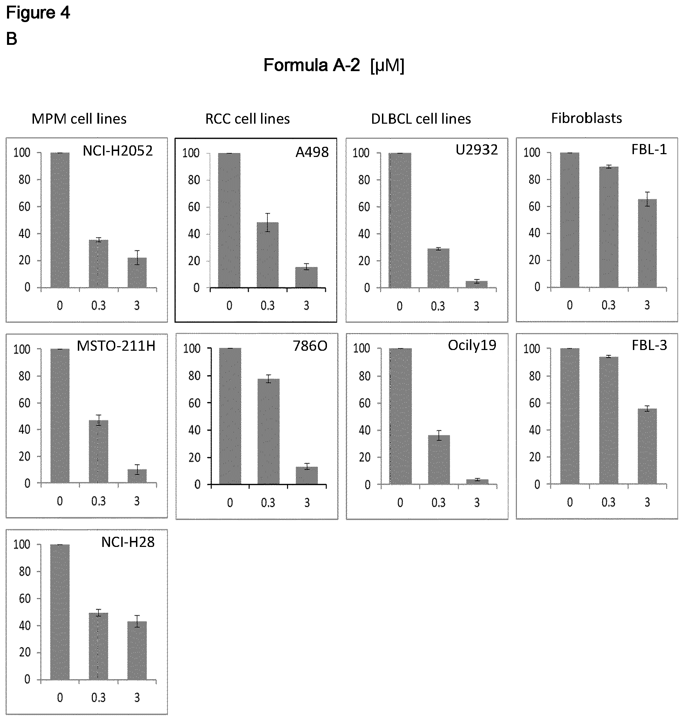

As shown in Figs. 1 to 6, the inventors found that indolyl-chalcone compounds of *Formulas A-1* and *A-2* are highly effective in reducing the viability (i.e. in killing) MPM, RCC and DLBCL cells *in vitro while* only moderately affecting the viability of healthy non-tumor cells (Fig. 4) and that they are superior in reducing the viability of MPM and DLBCL cells compared to standard MPM or DLBCL therapeutics (Figs. 11 and 12).

Therefore, the present invention relates to indolyl-chalcone compounds of *Formula A* for use in the treatment of malignant pleural mesothelioma (MPM), renal cell carcinoma (RCC) or diffuse large B-cell lymphomas (DLBCL). Specifically, the invention relates to a pharmaceutical composition comprising a compound of 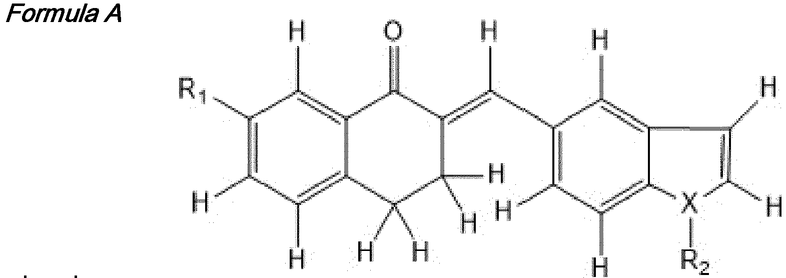 wherein:
X is a heteroatom;
R₁ is halo; and
R₂ is a C₁-C₁₀ alkyl,
for use in the treatment of malignant pleural mesothelioma (MPM), renal cell carcinoma (RCC) or diffuse large B-cell lymphomas (DLBCL).

Generally, the compound of Formula A is a compound of ***Formula A-1*** 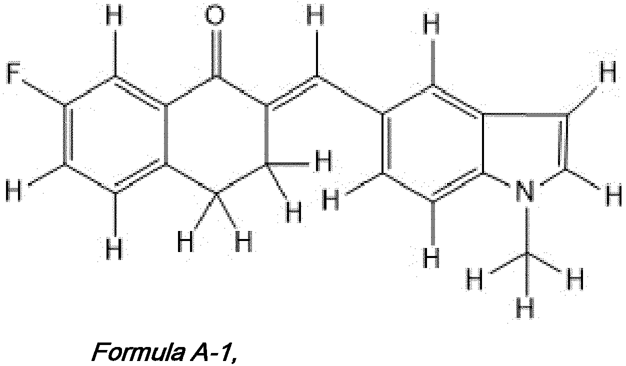 or a compound of ***Formula A-2***

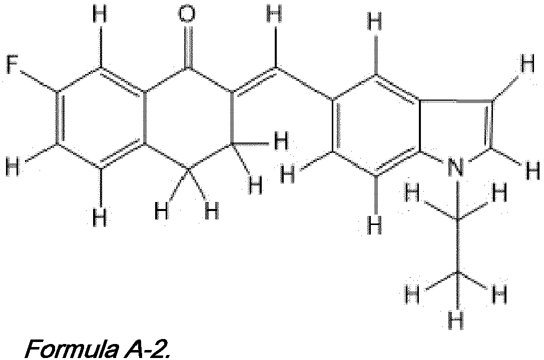

Given the high occurrence of resistance in MPM, RCC and DLBCL to the typical treatment regimens, the identification of compounds of *Formula A,* specifically of *Formula A-1 orA-2,* as being effective in mediating MPM, RCC and DLBCL cell death, allows for further treatment options.

As such, in some embodiments, the treatment of MPM, RCC or DLBCL is treatment of MPM, RCC or DLBCL partially or fully resistant to other chemotherapeutic agents and/or immunotherapeutic agents.

In relation to MPM, this means that, typically, the treatment is treatment of MPM partially or fully resistant to:
(a) platinum-based chemotherapeutic agents; and/or
(b) folate antimetabolite chemotherapeutic agents.
Specifically, the treatment is treatment of MPM partially or fully resistant to a combination of chemotherapeutic agents comprising:
- cisplatin and pemetrexed; and/or
- cisplatin and raltitrexed.

In relation to RCC, this means that, typically, the treatment is treatment of RCC partially or fully resistant to:
(a) therapeutic agents inhibiting mammalian/mechanistic target of rapamycin (mTOR); and/or therapeutic agents inhibiting vascular endothelial growth factor (VEGF) mediated formation of blood vessels, preferably via inhibition of tyrosine kinase activities and/or immunotherapeutic agents directly blocking VEGF; and/or
(b) immunotherapeutic agents promoting an immune reaction against said RCC.

Specifically, the treatment is treatment of RCC partially or fully resistant to:
- mTOR inhibitors such as everolimus and/or temsirolimus; and/or tyrosine kinase inhibitors such as sunitinib and/or axitinib; and/or
- monoclonal antibodies such as avelumab, ipilimumab, nivolumab and/or bevacizumab.

In relation to DLBCL, this means that, typically, the treatment is treatment of DLBCL partially or fully resistant to a combination of chemotherapeutic and immunotherapeutic agents such as a combination comprising several chemotherapeutic agents, a steroid and a targeted immunotherapeutic agent.

Specifically, the treatment is treatment of DLBCL partially or fully resistant to a combination of chemotherapeutic and immunotherapeutic agents comprising rituximab, cyclophosphamide, doxorubicin hydrochloride, vincristine and prednisolone.

Of course, new effective treatment options with a low side effect profile (i.e. which only cause mild or few side effects), also allow for new, first-line combination treatments. In particular, compositions comprising the compounds of *Formula A,* preferably of *Formula A*-*1* and/or *A-2,* can be used to replace chemotherapeutic agents, against which resistances are developed most frequently during current standard treatment regimens and/or which have a high side effect profile.

As such, in the context of MPM, the pharmaceutical composition of the present disclosure can be used for combination treatment of MPM. Namely, the compositions comprising the compounds of *Formula A,* preferably *A*-*1* and/or *A-2,* can be for use in combination treatment of MPM also comprising the administration of platinum-based chemotherapeutic agents and/or folate antimetabolite chemotherapeutic agents.

In the context of RCC, the pharmaceutical composition of the present disclosure can be used for combination treatment of RCC. Namely, the compositions comprising the compounds of *Formula A,* preferably *A*-*1* and/or *A-2,* can be for use in combination treatment of RCC also comprising the administration of chemotherapeutic agents inhibiting vascular endothelial growth factor mediated (VEGF-mediated) formation of blood vessels and/or immunotherapeutic agents promoting an immune reaction against the RCC.

In the context of DLBCL, the pharmaceutical composition of the present disclosure can be used for combination treatment of DLBCL. Namely, the compositions comprising the compounds of *Formula A,* preferably *A*-*1* and/or *A-2,* can be for use in combination treatment of DLBCL also comprising the administration of several further chemotherapeutic agents, a steroid and a targeted immunotherapeutic agent.

The inventors further show that the therapeutic efficacy of the chalcone compounds of *Formula A*-*1* and *A-2* are independent of stathmin (STMN1) and/or collapsin response mediator protein-2 (CRMP2) protein expression.

Both STMN1 and CRMP2 are major tubulin and mitosis regulators. Specifically, STMN1 is a microtubule destabilizing protein that plays an important role in cell cycle progression, segregation of chromosomes, cell motility and survival. STMN1 acts directly on tubulin heterodimers, preventing the formation of microtubules. Phosphorylation of STMN1 reduces the affinity between STMN1 and tubulin heterodimers, thus enabling tubulin polymerization.

Microtubules form part of the cytoskeleton and are the major constituents of mitotic spindles. During mitosis, accurate microtubule dynamics is necessary for successful cell cycle progression and tight regulation of STMN1 phosphorylation is crucial for this process. In addition, STMN1 has a "relay" function in signal transduction, integrating multiple signaling pathways: PI3K, MAPK, cAMP, cyclin-dependent kinase (CDK), and Ca^{2+/}Calmodulin signaling control the stability and phosphorylation status of STMN1. Up-regulation of STMN1 in cancer cells promotes cell proliferation, migration, metastasis and resistance to chemotherapy. Knockdown of STMN1 significantly reduces tumor growth and metastasis and induces apoptosis in preclinical experiments. A first study on STMN1 expression in MPM, detected increased protein levels in 7 of 8 MPM tumor samples. Knockdown of STMN1 in MPM cells led to inhibition of cell proliferation and motility.

Collapsin response mediator protein-2 (CRMP2), also known as DPYSL2, is another microtubule-associated protein that is deregulated in cancer cells. CRMP2 binds to tubulin dimers and promotes microtubule assembly by adding tubulin dimers to the growing microtubule. Several signaling pathways, e.g. Sema3A, Rho, PI3K/AKT signaling, regulate CRMP2 phosphorylation and thus its affinity to bind tubulin. CRMP2 participates in neuronal growth, vesicle transport, migration and mitosis. Elevated expression of nuclear phosphorylated CRMP2 has been implicated in cancer progression. CRMP2 silencing induced tumor cell apoptosis, pointing to CRMP2 as a new potential target for cancer therapy.

In accordance with the present disclosure, use of a compound of ***Formula A*** 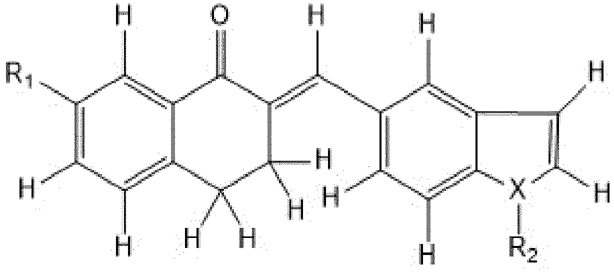 wherein:
X is a heteroatom;
R₁ is halo; and
R₂ is a C₁-C₁₀ alkyl,
for disturbing the polymerization of tubulin molecules is also provided.

Furthermore, use of a compound of ***Formula A*** 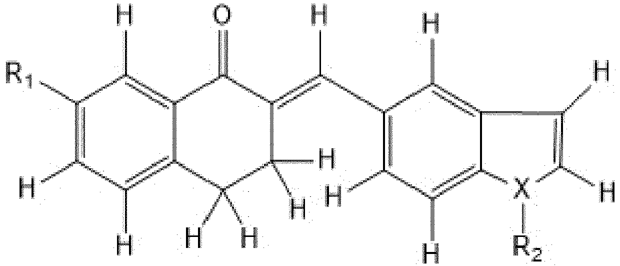 wherein:
X is a heteroatom;
R₁ is halo; and
R₂ is a C₁-C₁₀ alky,
for inhibiting formation of a functional spindle apparatus is provided.

Importantly, the compounds of *Formula A,* preferably *A*-*1* and/or *A-2,* can be used for both disturbing the polymerization of tubulin molecules and inhibiting formation of a functional spindle apparatus independently of STMN1 and/or CRMP2 protein expression. Such uses can be helpful in *in vitro* investigations of cancer-types and cell lines negative for these activities such as to obtain pre-clinical data of further combination therapy regimens, but also to better understand the interplay of factors and proteins necessary for appropriate or aberrant tubulin polymerization or spindle apparatus formation. Such fundamental research forms the basis for the identification of new targets of cancer therapies.

Furthermore, the pharmaceutical composition of the present disclosure can be used for treatment of MPM, RCC or DLBCL irrespective of the tumor cells' expression of STMN1 and/or CRMP2.

### Examples

The invention is further described by the following non-limiting Examples, in which the inventors have analyzed indolyl-chalcones of ***Formula A*** 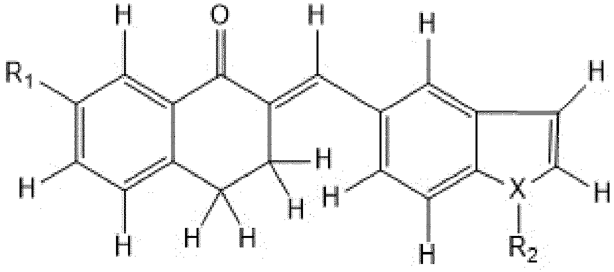 wherein:
X is a heteroatom;
R₁ is halo; and
R₂ is a C₁-C₁₀ alky,
for their antitumor activity against MPM, RCC and DLBCL cells. In particular, the impact of these compounds on cells displaying resistance to the currently established and recommended chemotherapeutic treatment regimens as well as the compounds' influence on microtubule dynamics and microtubule regulators STMN1 and CRMP2 was investigated.

### Materials and methods

### Cell lines

MPM cell lines NCI-H28 (epithelioid MPM) and NCI-H2052 (sarcomatoid MPM) were purchased from LGC Standards GmbH (Wesel, Germany), MPM cell line MSTO-211H (biphasic MPM) was purchased from DSMZ (Deutsche Sammlung von Mikroorganismen und Zellkulturen, Braunschweig, Germany). All MPM cell lines were cultured in RPMI medium supplemented with 10% FBS (Biochrom AG, Berlin, Germany).

RCC cell lines 7860 and A498 were purchased from CLS Cell Lines Service (Eppelheim, Germany) and cultured in RPMI medium supplemented with 10% FBS.

DLBCL cell line U2932 and OciLy19 were purchased DSMZ (Deutsche Sammlung von Mikroorganismen und Zellkulturen, Braunschweig, Germany) and cultured in RPMI medium supplemented with 20% FBS.

Normal fibroblast cells were gained from reactive lymph node resections from two individuals as described in Staiger et al. (2017; Leukemia Lymphoma 58, 1922-1930) and cultured in RPMI supplemented with 20% FBS.

### Antibodies and reagents

Antibodies to detect STMN1 (D1Y5A), phospho-STMN1 (Ser16), CRMP2 (D8L6V), phospho-CRMP2 (Thr514), PARP (46D11), c-Jun (60A8), phospho-c-Jun (Ser63), AKT (C67E7), phospho-AKT (Ser473, D9E), ERK1/2 (137F5), phospho-ERK1/2 (Thr202/Tyr204), GAPDH (D16H11)) were from Cell Signaling (Danvers, MA, USA). Anti-β-Actin (AC-15) and Anti-α-Tubulin (DM1a) were from Sigma-Aldrich (München, Germany). Alexa488-coupled anti-mouse antibody was from Fisher Scientific (Schwerte, Germany). Paclitaxel, cisplatin, pemetrexed, doxorubicin and vincristine were purchased from Selleckchem (Houston, TX, USA).

### Compounds of Formula A

Indolyl-chalcone compounds of *Formulas A-1 and A-2* were synthesized by the aldol condensation between a tetralone and an indole aldehyde (as previously described in Wegiel, B., Wang, Y., Li, M., Jernigan, F. & Sun, L.; Cell Cycle 15, 1288-1294 (2016), the disclosure of which is incorporated by reference herein). The compounds were diluted in DMSO at 10 mM and then diluted in culture medium at 0.01-10 µM final concentrations.

### Immunohistochemistry

IHC was accomplished on sections of Formalin-Fixed Paraffin- FFPE-embedded cell lines using conventional DAB staining on a semi-automated autostainer (LabVision 720, Thermo Fisher Scientific, Waltham, MA, USA). After heat-induced epitope retrieval at pH6, anti-Stathmin (1:100) or anti-CRMP2 (1:100) was incubated for 30 minutes.

### Cell viability assay

MPM and DLBCL cells were seeded into 96-well plates (4×10³ cells/100µl) and incubated with compounds of *Formula A*-*1* and *Formula A-2*(0.01-10 µM) or DMSO for 72 h. After addition of 5 mg/ml MTT (3-(4,5-dimethylthiazol-2-yl)-2,5-diphenyl tetrazolium bromide) to each well, the cells were further incubated for 2 h. The produced formazan blue was dissolved in 15% SDS in dimethylformamide-water (1:1), and the absorbance was measured at 550 nm using an EnSpire Multimode Plate Reader 2300 (PerkinElmer). Percent viability was calculated by normalizing absorbance values to those from cells grown in media without drug after background subtraction. IC₅₀ values were calculated by non-linear regression analysis (Prism Software, GraphPad).

RCC cells were seeded into 96-well plates (4×10³ cells/100µl) and incubated with compounds of *Formula A*-*1* and *Formula A-2* (0.01-3 µM) or DMSO in the presence of the RealTime-Glo^{™} MT Cell Viability Assay (Promega). After 72h incubation luminescence was measured using an EnSpire Multimode Plate Reader 2300 (PerkinElmer). Percent viability was calculated by normalizing luminescence values to those from cells grown in media without drug after background subtraction. IC₅₀ values were calculated by non-linear regression analysis (Prism Software, GraphPad).

### Cell death

For investigation of apoptosis and necrosis, cells were seeded at densities of 1×10⁵ cells in 1 ml media into 24-well plates and incubated for 24 hours at 37°C. Then, cells were treated with compounds of *Formula A*-*1* and *Formula A-2* (500 nM) or DMSO (0.005%) and incubated for a further 24 h. The cells were then washed and harvested using accutase. Finally, the cells were collected and stained using Annexin V-APC (Immunotools, Friesoythe, Germany) and 25 µg/ml PI (propidium iodide) for 10 min. Cells were analyzed by a BD FACSLyric Flow Cytometery System (BD, Heidelberg, Germany). Phase-contrast microscopy was used to investigate morphological changes. Cells (1×10⁵ cells/ml) were seeded into 24-well plates and incubated with *Formula A-1* (300 nM) or *Formula A*-2(300 nM) for 24 h. Cells were imaged using an CKX41 inverted microscope and Olympus Cell^F (Olympus Life Science, Tokyo, Japan).

### Tubulin polymerization assay

A tubulin polymerization assay kit (part# BK006P, Cytoskeleton, Denver, CO, USA) was used to address the direct effects of chemical compounds on tubulin polymerization dynamics. A porcine >99% pure tubulin preparation (3 mg/ml) free of microtubule-associated proteins in 80 mM PIPES pH 6.9, 2 mM MgCl₂, 0.5 mM EGTA, 1 mM GTP, and 10.2% glycerol was incubated with *Formula A*-*1* (100 µM), *Formula A-2*(100 µM), paclitaxel (10 µM), or buffer. Microtubule polymerization was initiated by tubulin addition; changes in microtubule turbidity were measured kinetically at 340 nm at 35°C (EnSpire Multimode Plate Reader 2300, PerkinElmer).

### Immunofluorescence

Analysis by immunofluorescence microscopy was performed as described in Schneider et al. (2007; J. Biol. Chem. 282, 29273-29283). Briefly, 1×10⁵ cells/cm² cells were seeded on #1 coverslips and incubated with compounds of *Formula A*-*1* and *Formula A-2* (100 nM) or DMSO (0.001%) for 24 h. Then, cells were fixed with ice-cold methanol/acetone (1:1) for 20 min and subsequently incubated in immunofluorescence (IF) buffer (4% bovine serum albumin, 0.05% saponin in PBS) for 1 h. Coverslips were incubated in IF buffer containing α-Tubulin antibody (1:500) overnight at 4°C. Thereafter, cells were washed twice in IF buffer and incubated with Alexa488-coupled anti-mouse antibody (1:400). DNA was detected by incubation with Hoechst 34580 (100 ng/ml). Fluorescence images were captured using a Leica TCS SP8 confocal laser scanning microscope equipped with an HCX PL APO × 63 immersion objective (Leica, Wetzlar, Germany) using excitation at 405 nm and 488 nm at 600 Hz scan speed with line average and identical laser intensity/amplification of detectors for each series. Images are projections of recorded z-stacks of various dimensions.

### Cell cycle analysis

Cells were seeded at densities of 1×10⁵ cells in 1 ml media into 24-well plates and incubated for 24 hours at 37°C. Then, cells were treated with compounds of *Formula A-1* and *Formula A*-2(300 nM), or DMSO (0.003%) and incubated for a further 14 h. The cells were then washed and harvested using accutase. Finally, the cells were collected and stained using 50 µg/ml PI (propidium iodide) and 50 µg/ml RNase A for 25 min. The proportion of PI stained cells was determined by using a BD FACSLyric Flow Cytometery System.

### Immunoblotting

Snap-frozen cells were homogenized in lysis buffer (50mM TRIS-HCl pH 7.6, 250mM NaCl, 5mM EDTA, 0,1% Triton X-100) containing complete protease inhibitor cocktail and PhosSTOP phosphatase inhibitor (Roche, Mannheim, Germany) by sonication. Immunoblotting was performed as previously described. Antibodies were used at 1:1.000 dilution, except antibodies against CRMP2, phospho-Stathmin (1:500); phospho-AKT, phospho-ERK1/2,(1:2.000); β-Actin (1:10.000). Luminescent signals were detected with the digital gel documentation system Stelle3200 (Raytest, Straubenhardt, Germany) and quantified using imaged 152-win-java8 software (National Institutes of Health, USA).

### STMN1knockdown

Cells were transfected with either 30 nM non-targeting control siRNA (ON-TARGETplus Non-targeting pool, Horizon Discovery, Cambridge, United Kingdom) or 30 nM human pooled STMN1 siRNAs (ON-TARGETplus Human STMN1 siRNA, Horizon Discovery) using Lipofectamine 3000 (Thermo Fisher Scientific). Twenty-four hours after transfection, cells were treated with *Formula A*-*1* (0.3-3µM) or *Formula A-2* (0.3-3 µM) in 96-well plates for 72 h and subsequently subjected to MTT assay. To confirm successful knockdown, STMN1 expression was analyzed by immunoblotting.

### Statistical analysis

The statistical tests were done using GraphPad Prism 5.00. The test used, number of samples and significance are indicated in the respective figure legends.

### Example 1

### Compounds of Formula A-1 and Formula A-2 decrease MPM, RCC and DLBCL viability and proliferation

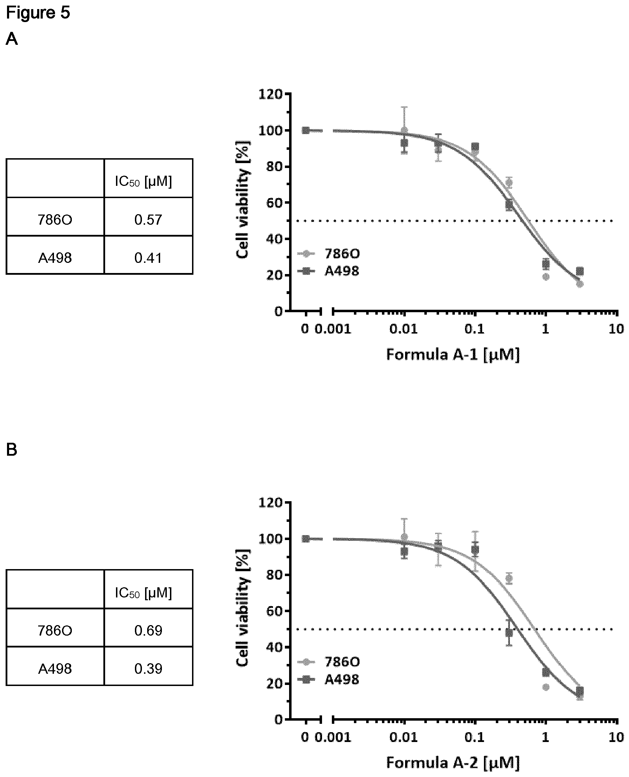

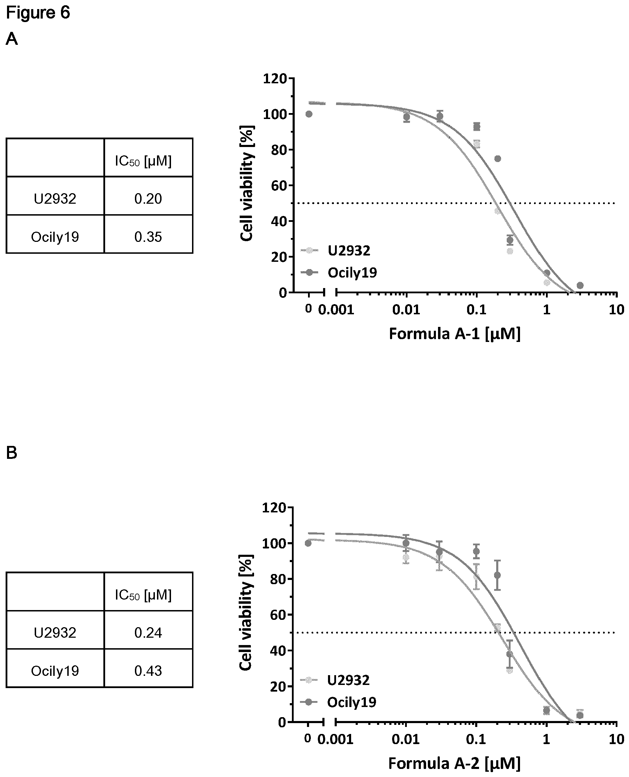

The cytotoxic effect of indolyl-chalcones of *Formula A*-*1* and *Formula A-2* on:
- three MPM cell lines - NCI-H28 (epithelioid MPM), NCI-H2052 (sarcomatoid MPM) and MSTO-211H (biphasic MPM) - Figs. 1 to 4;
- two RCC cell lines - 7860 and A498 - Figs. 4 and 5; and
- two DLBCL cell lines - U2932 and OciLy19 - Figs. 4 and 6.

Cells were exposed to increasing concentrations (0.01 to 10 µM) of *Formula A*-*1* and *Formula A-2*for 72 hours, after which cell viability was assessed by an MTT assay (MPM, DLBCL) or the RealTime-Glo^{™} MT Cell Viability Assay (RCC). Both compounds of *Formula A*-*1* and *Formula A-2* strongly impaired cell viability and proliferation in all cell lines (Figs. 1 to 6).

From the combined data for the cell lines of the respective tumor entities, the below IC₅₀ values for the compounds of *Formula A*-*1* and *A-2* were calculated:

| Tumor | IC₅₀ | |
|---|---|---|
| | *Formula A*-*1* | *Formula A-2* |
| MPM | 0.20-0.34 µM | 0.14-0.33 µM |
| RCC | 0.41-0.57 µM | 0.39-0.69 µM |
| DLBCL | 0.20-0.35 µM | 0.24-0.43 µM |

Non-linear regression analysis of log(inhibitor) versus response was used for IC₅₀ calculation.

In addition, the impact of *Formula A*-*1* and *Formula A-2* on the viability and proliferation of non-tumor cells, i.e. fibroblasts from two individuals, was assessed. MTT assay did not indicate reduced viability of fibroblasts after 72 h incubation with 0.3 µM *Formula A-1* or *Formula A-2,* a concentration that diminished viability of MPM, RCC and DLBCL cell lines (Fig. 4). At 3 µM, the effects of both compounds of *Formula A-1* and *Formula A-2* on fibroblasts were comparable to the responses obtained with 0.1 to 0.3 µM *Formula A-1* or *Formula A-2* in MPM cells, 0.3 µM *Formula A-1* or *Formula A-2* in RCC cells, 0.2 µM *Formula A-1* or *Formula A-2* in DLBCL cells indicating that the impact of *Formula A-1 and Formula A-2* on cell viability was at least ten times stronger on tumor cells than on healthy non-tumor cells.

### Example 2

### Compounds of Formula A-1 and FormulaA-2induce apoptotic cell death in MPM cells

To identify the mechanism of cell death induced by indolyl-chalcone compounds of *Formula A,* MPM cells were labelled with Annexin V and propidium iodide (PI) and analyzed by flow cytometry after 24 hours of treatment with *Formula A*-*1* (500 nM), *Formula A-2(500* nM) or DMSO. Early apoptotic cells bind Annexin V but exclude the membrane-impermeable DNA stain PI (Annexin V positive/PI negative cells), while late-stage apoptotic cells stain positively for Annexin V and PI (Annexin V positive/PI positive cells).

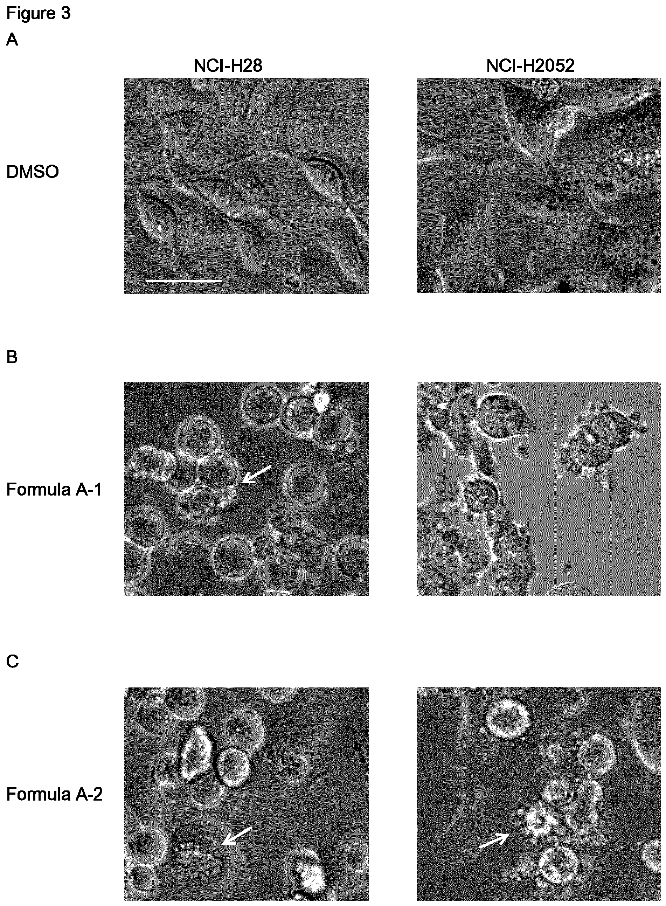

It was found that *Formula A-1* and *Formula A-2* induce the accumulation of Annexin V positive/PI negative cells and of Annexin V positive/PI positive cells indicating apoptotic cell death in NCI-H2052 and NCI-H28 cells (Fig. 2A and 2B). Induction of apoptosis was confirmed by apoptotic membrane blebbing (Fig. 3) and cleavage of the caspase-substrate PARP1 (poly(ADP-ribose) polymerase 1) detected in NCI-H28 cells (Fig. 2C) following exposure to *Formula A-1* (1 µM) or *Formula A-2* (1 µM) for 24 hours.

### Example 3

### Indolyl-chalcone compounds of Formula A disturb tubulin polymerization and induce cell cycle arrest

To dissect the effects of compounds of *Formula A-1* and *Formula A-2* on the cellular microtubule network and mitotic spindles in MPM cells, microtubules were visualized after 24 hours of *Formula A-1* and *Formula A*-2treatment using an α-tubulin specific antibody. Control DMSO-treated cells have a normal fine-structured filamentous tubulin network and regular mitotic spindles. In contrast, NCI-H2052 and NCI-H28 cells exposed to *Formula A-1* or *Formula A*-2display an aberrant microtubule network featuring shortened, branched tubulin fibers and a lack of long fibers (Fig. 7A and 7B). In both cell lines, abnormal spindle morphology was accompanied by perturbed chromosomal arrangement in (pro)metaphase and an accumulation of mitotic nuclei (Fig. 7A). The inventors found that the cell cycle was arrested in G2/M phase in MPM cells after 14 hours of *Formula A-1* and *Formula A-2* treatment (Fig. 7C).

To determine whether the influence of compounds of *Formula A-1* and *Formula A-2* on microtubule formation was due to on-target activity, the direct effect of chalcone compounds of *Formula A* on tubulin polymerization rates was examined by incubation of purified porcine tubulin with *Formula A-1* or *Formula A-2* and comparison of the results with those of paclitaxel, a well-established microtubule-stabilizing agent. Microtubule formation was measured kinetically at an absorbance of 340 nm. In the presence of paclitaxel, the kinetics of tubulin polymerization is enhanced and the polymerization equilibrium shifts towards polymerized tubulin due to microtubule stabilization (Fig. 7D). *Formula A*-*1* and *Formula A-2* have a similar effect on the kinetics, but not on the equilibrium of tubulin polymerization seen after 10 min indicating that compounds of *Formula A*-*1* and *Formula A-2* promote tubulin polymerization, but not microtubule stabilization (Fig. 7D). These data suggest that direct interaction with tubulin contributes to microtubule disturbance inferred by the chalcone compounds of *Formula A,* which ultimately leads to aberrant spindle assembly and mitotic arrest.

### Example 4

### STMN1 and CRMP2 are targets of chalcone compounds of Formula A

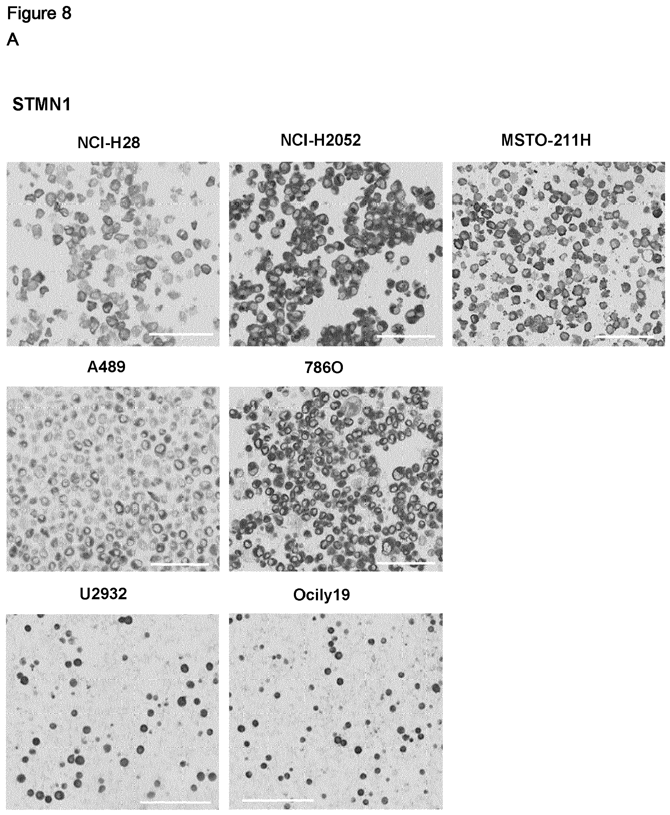

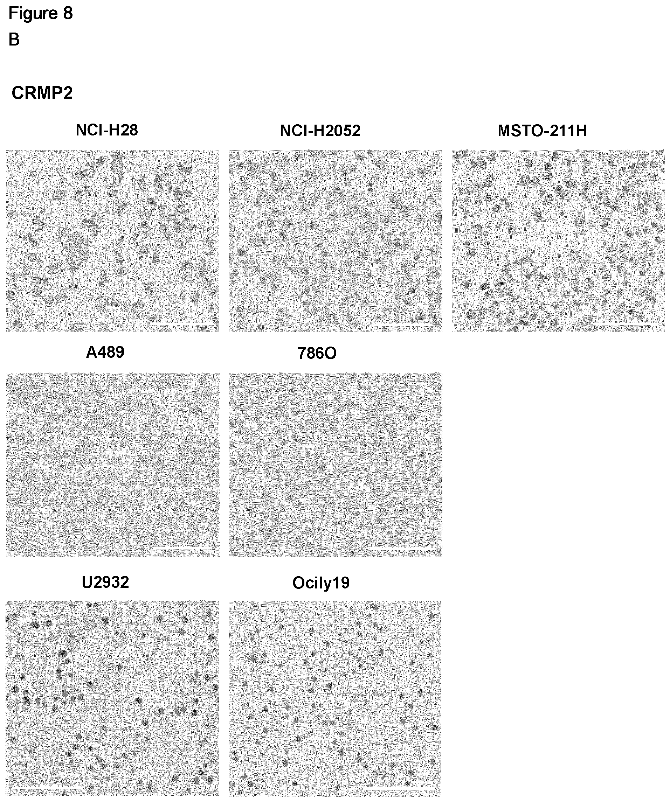

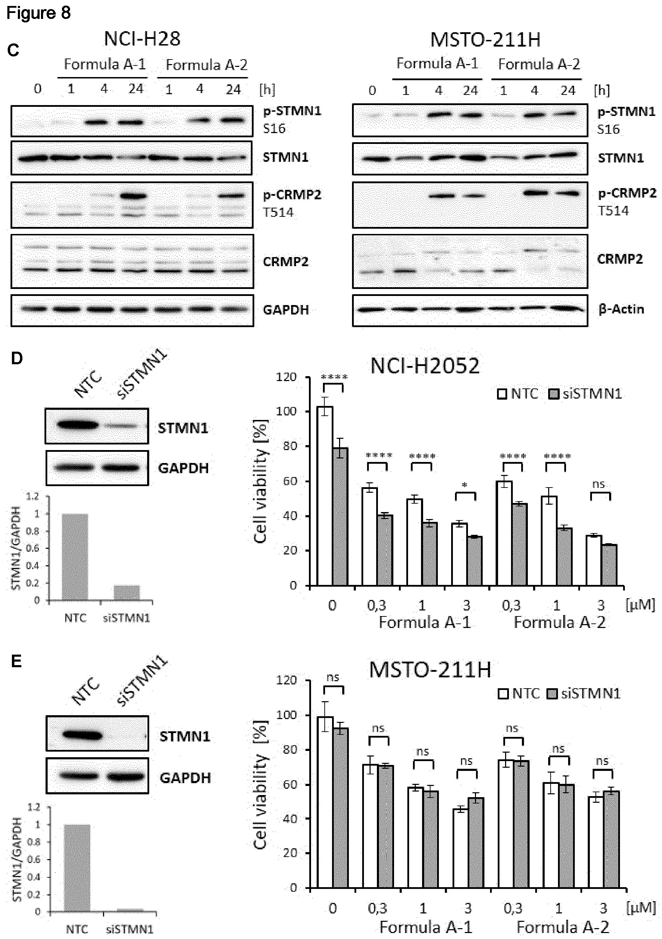

Next, the role of STMN1 and CRMP2, two major tubulin and mitosis regulators, in mediating the anti-tumor effects of compounds of *Formula A*-*1* and *Formula A-2* in MPM cells was evaluated. All MPM (NCI-H28, NCI-H2052, MSTO-211H), RCC (A489, 7860) and DLBCL cell lines (U2932, Ocily19) expressed STMN1 protein, i.e. all cell lines are positive for STMN1 (Fig. 8A). Significant amounts of CRMP2 are only expressed in MPM cell lines NCI-H28 and MSTO-211H, whereas all other tumor cell lines are negative for CRMP2: NCI-H2052 and A489 have low-level CRMP2 expression (faint CRMP2 immunohistochemistry staining) and 7860, U2932 and Ocily19 do not express CRMP2 (Fig. 8B).

When NCI-H28 and MSTO-211H cells are treated with 1 µM *Formula A-1* or 1 µM *Formula A-2* for 1 to 24 hours, phosphorylation of STMN1 and CRMP2 is effectively enhanced in a time-dependent manner and reaches hyper-phosphorylation levels at 24 hours (Fig. C).

To determine whether the activity of compounds of *Formula A*-*1* and *Formula A-2* is dependent upon STMN1, the consequences of reduced STMN1 protein expression on the efficacy of chalcone compound therapy was examined by knocking down STMN1 in cell lines MSTO-211H (positive for STMN1 and CRMP2) and NCI-H2052 (positive for STMN1, negative for CRMP2). Knockdown of STMN1 by siRNA was confirmed by immunoblotting (Fig. 8D, 8E). The knockdown significantly reduces the viability of NCI-H2052 cells, while MSTO-211H cells remain unaffected. In NCI-H2052 cells, cell death in response to *Formula A*-*1* and *Formula A-2* is accelerated in the absence of STMN1 (Fig. 8D). In MSTO-211H cells, STMN1 knockdown does not have a significant impact on the therapeutic effect of *Formula A*-*1* and *Formula A-2* (Fig. 8E). Taken together, these findings suggest that the activity of compounds of *Formula A-1* and *Formula A-2* is associated with STMN1 and CRMP2 hyper-phosphorylation, but is not dependent upon STMN1 activity.

### Example 5

### Chalcone compounds of Formula A affect intracellular signaling in MPM cells

To gain a more detailed insight into cellular pathways affected by chalcone compounds of *Formula A,* the effects of compounds of *Formula A*-*1* and *Formula A-*2on proteins involved in stress response and apoptosis induction were investigated. Upon 4-hour *Formula A-1* or *Formula A-2* exposure, an activation of JNK/c-Jun, p38/MAPK and AKT signaling pathways is observed, with cell-line dependent differences (Fig. 9, Fig. 10): Phospho-c-Jun (S63) is enhanced in both cell lines, in NCI-H28 cells associated with induction of c-Jun expression. In NCI-H28 cells, both compounds of *Formula A*-*1* and *Formula A-2* induce a time-dependent increase in phospho-ERK and phospho-AKT levels (Fig. 9A). In MSTO-211H cells, chalcone compound treatment reduces phospho-ERK, but does not affect AKT phosphorylation (Fig. 9B).

Taken together, the inventors' data suggest that chalcone compounds of *Formula A* act via deregulation of tubulin-associated phospho-proteins (STMN1, CRMP2) and affect key regulators of stress response, cell survival and apoptosis induction (c-Jun, AKT, ERK1/2).

### Example 6

### Chalcone compounds of Formula A are more effective than standard chemotherapeutics in MPM cells

MPM cell lines NCI-H28, NCI-H2052 and MSTO-211H were exposed to the components of the standard therapy regimen cisplatin (at concentrations of 0, 0.3, 1, 3, 5 and 10 µM) and pemetrexed (at concentrations of 0, 0.01, 0.03, 0.1, 0.3, 1, 3 and 10 µM) for 72 h (Fig. 11A). For comparison, NCI-H28, NCI-H2052 and MSTO-211H cells were incubated with *Formula A-1* or *Formula A-2* (at concentrations of 0, 0.01, 0.03, 0.1, 0.3, 1, 3 and 10 µM) for 72 h (Fig. 11B). Cell viability was assessed by an MTT assay in independent triplicate experiments.

The compounds of *Formula A* impair cell viability efficiently in all three MPM cell lines. Furthermore, the compounds of *Formula A* are more efficient in killing MPM cells than the standard MPM chemotherapeutics:
- NCI-H28 cells are partially resistant to both standard chemotherapeutics, i.e. cells do not respond to cisplatin until high concentrations of 5 to 10 µM are applied, which achieve a reduction of cell viability of 33 to 48%; reduction in cell viability plateaus at 52 to 55% at concentrations of 1 to 10 µM pemetrexed.
- In comparison, reduction of cell viability in NCI-H28 cells was 59 to 71% (at concentrations of 1 to 10 µM *Formula A-1*) and 58 to 68% (at concentrations of 1 to 10 µM *Formula A-2*)*.*

### Example 7

### Chalcone compounds of Formula A are more effective than standard chemotherapeutics in DLBCL cells

DLBCL cell line U2932 was exposed to the components of the R-CHOP standard therapy regimen doxorubicin (at concentrations of 0, 1, 3 and 10 µM) and vincristine (at concentrations of 0, 1, 3, 10, 30 and 100 nM) for 16 h. Immediately thereafter, the cells were cultured without exposure to either agent for further 24 h either under "stromal protection" or not, i.e. either in the presence or absence of stromal cells (HS-5; Fig. 12A). Co-culture of DLBCL cells with HS-5 cells reduces the effect of R-CHOP components doxorubicin and vincristine substantially. For comparison, U2932 cells were incubated with *Formula A*-*1* or *Formula A-2* (at concentrations of 0, 0.2, 0.3, 1 and 3 µM) for 72 h either under "stromal protection" or not, i.e. either in the presence or absence of stromal cells (HS-5; Fig. 12B). Cell death induction was measured by Annexin V staining and flow cytometry analysis in independent duplicates.

The compounds of *Formula A* induce cell death efficiently in DLBCL cells when cultured without stromal protection, i.e. in the absence of stromal cells. Furthermore, the compounds of *Formula A* are more effective in killing DLBCL cells even in a stromal microenvironment than the standard therapeutics:
- stromal-induced efficacy reduction was only 16 to 25% (at concentrations of 1 to 3 µM *Formula A-1*) and 9 to 25% (at concentrations of 1 to 3 µM *Formula A-2*)
- compared to a reduction in efficacy of 42 to 45% (at concentrations of 1 to 10 µM doxorubicin) and 40 to 52% (at concentrations of 10 to 100 nM vincristine).

### DISCUSSION

The present disclosure shows that indolyl-chalcones of *Formula A* act as microtubule-targeting agents to induce mitotic arrest and apoptosis in MPM, RCC and DLBCL cells.

Indolyl-chalcones of *Formula A*-*1* and *Formula A-2* are both toxic to three MPM cell lines, two RCC cell lines and two DLBCL cell lines at sub-micro molar concentrations.

In addition to the demonstration of anti-tumor efficacy, the present disclosure provides new insights into the mechanism of action of indolyl-chalcones compounds of *Formula A* and novel uses thereof. Using a tubulin polymerization assay, it was shown that compounds of *Formula A*-*1* and *Formula A-2*directly interact with tubulin molecules and accelerate tubulin polymerization. Indolyl-chalcones compounds of Formula A cause apoptosis as a result of disruption of mitotic progression due to abnormal spindle morphology (Fig. 10). Consistently, an accumulation of nuclei arrested in G2/M phase is observed.

Treatment with indolyl-chalcones compounds of *Formula A* also results in activation of proteins involved in stress response, cell survival and apoptosis, including transcription factor (c-Jun) and kinases (AKT and ERK). Activation of c-Jun has been demonstrated to act pro-apoptotic in response to the microtubule stabilizing agent paclitaxel. In addition, several agents targeting microtubules, such as paclitaxel, are known to induce apoptosis via inhibition of AKT/ERK signaling. Consistently, apoptosis induction upon treatment with indolyl-chalcones compounds of *Formula A* is associated with ERK inhibition in MSTO-211H cells. In NCI-H28 cells, phospho-AKT and phospho-ERK are enhanced upon treatment with indolyl-chalcones compounds of *Formula A,* which however does not prevent apoptosis induction. As such, and without wanting to be bound by theory, apoptosis of MPM, RCC and DLBCL cells in response to treatment with indolyl-chalcones compounds of *Formula A* appears to be mediated by activation of pro-apoptotic signaling (c-Jun) and - depending on the cellular background - inhibition of ERK thus overriding pro-survival signaling by c-Jun, ERK and AKT (Fig. 10).

Unlike conventional microtubule-targeting agents, small-molecule indolyl-chalcones compounds of *Formula A* are not only able to interact with tubulin, but can also bind to various enzymes including oxidoreductases, transcription factor NF-κB, and pro-apoptotic proteins (e.g. caspases-3 and -8), depending on their substituents. Chalcone interaction with tubulin and enzymes is mediated by the formation of hydrogen bonds and hydrophobic interactions between the chalcone-substituents and tubulin or amino acids present in the enzyme active sites.

The inventors observed hyper-phosphorylation of the microtubule regulators STMN1 and CRMP2 in response to treatment with indolyl-chalcones compounds of *Formula A*. Mechanistically, STMN1 and CRMP2 hyper-phosphorylation appears to be due to direct interaction of the indolyl-chalcones with phosphorylation sites of both phosphoproteins. However, it cannot be excluded that hyper-phosphorylation of STMN1 is an effect of forced tubulin polymerization as previously detected in Xenopus eggs by Küntziger et al. (2001; Mol. Biol. Cell 12, 437-448).

In any case, STMN1 and CRMP2 hyper-phosphorylation appears to add significantly to the direct effect of compounds of *Formula A*-*1* and *Formula A-2* on microtubule polymerization:
(1) CRMP2 stabilizes polymerized tubulin at the plus end of microtubules and phosphorylated CRMP2 loses its affinity for microtubule heterodimers. Hence, CRMP2 hyper-phosphorylation is thought to destabilize microtubules.
(2) STMN1 binds tubulin heterodimers, thus preventing the formation of microtubules. Phosphorylation of STMN1 reduces the affinity of STMN1 and tubulin heterodimers. Hence, STMN1 hyper-phosphorylation promotes tubulin polymerization.

Simultaneously reduced tubulin stability (pCRMP2) and augmented polymerization (pSTMN1) is expected to interfere with the accurate formation of microtubule structures that are necessary for successful cell cycle progression (Fig. 10).

Accordingly, MPM cells display an aberrant microtubule network consisting of shortened, branched tubulin fibers upon chalcone compound treatment. The activity of tubulin-associated proteins is, however, not necessarily restricted to microtubule polymerization. Instead, STMN1 is also involved in cell cycle control, cell survival and apoptosis, as component of diverse cancer signaling networks including PI3K/AKT, Bcl-2 and NF-κB signaling. Accordingly, chalcone compound-induced phosphorylation of STMN1 affects intracellular signaling and contributes to mitotic arrest and apoptosis. In support of this, it is noted that GDP366-induced phosphorylation of STMN1 is known to promote cell cycle arrest and apoptosis in acute leukemia (Carlos, J. A. E. G. et al. (2021); Invest. New Drugs 39, 1139-1149).

However, although hyper-phosphorylation of STMN1 and CRMP2 was a major effect of chalcone compound treatment, STMN1 and CRMP2 activity was not essential for anti-cancerous effects of compounds of *Formula A*-*1* and *Formula A-2.* Knockdown of STMN1 in MPM cells did not block the anti-tumor activity of *Formula A*-*1* and *Formula A-2.* Further, compounds of *Formula A*-*1* and *Formula A-2* had a similar efficacy in CRMP2-positive (NCI-H28, MSTO-211H) and CRMP2-negative tumor cells (NCI-H2052, A489, 7860, U2932, Ocily19). These findings indicate that compounds of *Formula A*-*1* and *Formula A-2* exert, at least, a dual mode of action: (a) in STMN1 and/or CRMP2-positive cells - via direct interaction with tubulin in concert with STMN1/CRMP2 inhibition; and (b) in STMN1/CRMP2-negative cells, where STMN1/CRMP2 are *per se* inactive - via direct interaction with tubulin only. Thus, compounds of *Formula A*-*1* and *Formula A-2* are efficient therapeutic agents suitable not only for STMN1/CRPM2-positive tumors.

While both compounds of *Formula A-1* and *Formula A-2* are toxic to all three MPM cell lines, one RCC cell line and two DLBCL cell lines at a concentration of 0.3 µM, the viability of normal (healthy) fibroblasts is not significantly affected at this concentration. At higher concentration (3 µM), fibroblasts are moderately affected, consistent with the fact that they do proliferate at sub-confluent densities. This finding suggests that a therapeutic window exists wherein lower concentrations of *Formula A-1* and *Formula A-2* decrease the viability of cancer cells, with minimal effects on healthy cells. Thus, compounds of *Formula A-1* and *Formula A-2* have advantages over the currently available microtubule-targeting agents, which often induce immunosuppression and neurotoxicity. Notably, chalcone compounds of *Formula A* are effective in epithelioid and sarcomatoid MPM cells, suggesting that compounds of *Formula A* are suitable for therapy of MPM irrespective of the cytological subtype.

Cytotoxic anticancer agents currently used for MPM, RCC and DLBCL therapy are often ineffective due to drug resistance mechanisms developed by the tumor cells. The protective effect of stromal microenvironment that promotes cancer cell survival and drug resistance further contributes to the difficulty in elimination of cancer cells *in vivo.* The majority (65-75%) of MPM tumors is resistant to the current standard therapeutics cisplatin and pemetrexed. Both compounds of *Formula A-1* and *Formula* A-2are more efficient in killing MPM cells than the standard therapeutics: MPM cells that are partially resistant to cisplatin and pemetrexed respond to both compounds of *Formula A,* which achieved a reduction of cell viability by 58 to 71%. Furthermore, the compounds of *Formula A* are more effective in killing DLBCL cells even in a stromal microenvironment than standard DLBCL therapeutics. Stromal protection is the main cause for therapy resistance and relapse observed in one third of all DLBCL patients within 6 to 12 months following treatment. Consistently, stromal protection caused a significant reduction (40 to 52%) in the efficacy of the standard therapeutics doxorubicin and vincristine in DLBCL cell line U2932. In contrast, stromal-induced efficacy reduction was only 9 to 25% when compounds of *Formula A-1* and *Formula A-2were* applied. Thus, compounds of *Formula A* act more efficiently against MPM and DLBCL cells than the standard therapeutics.

In summary, it is shown that the indolyl-chalcones of *Formula A* are highly effective anti-tumor agents against MPM, RCC and DLBCL cells, with only moderate effects on normal cells.

Both compounds of *Formula A-1* and *Formula A-2* induce MPM, RCC and DLBCL cell apoptosis through a dual mechanism of action: disruption of microtubule assembly and deregulation of microtubule-associated proteins STMN1 and CRMP2.

Importantly, the indolyl-chalcones of *Formula A* are still highly effective anti-tumor agents in cells, which are partially or fully resistant to the standard chemotherapeutic agents to be administered during the established and recommended treatments (i.e. cisplatin and pemetrexed in MPM or doxorubicin and vincristine in DLBCL).

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A pharmaceutical composition comprising a compound of ***Formula A*** 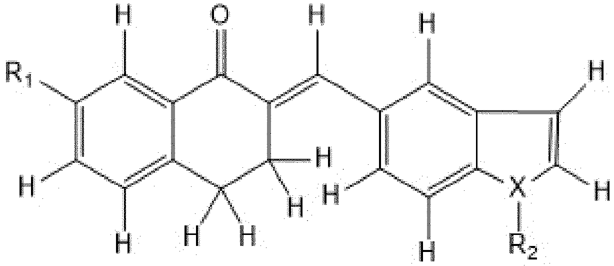 wherein:
X is a heteroatom;
R₁ is halo; and
R₂ is a C₁-C₁₀ alkyl,
for use in the treatment of malignant pleural mesothelioma (MPM), renal cell carcinoma (RCC) or diffuse large B-cell lymphomas (DLBCL).

2. The pharmaceutical composition for use according to claim 1, wherein said treatment is treatment of MPM, RCC or DLBCL partially or fully resistant to other chemotherapeutic agents and/or immunotherapeutic agents.

3. The pharmaceutical composition for use according to claim 2, wherein said treatment is treatment of MPM partially or fully resistant to:
(a) platinum-based chemotherapeutic agents; and/or
(b) folate antimetabolite chemotherapeutic agents.

4. The pharmaceutical composition for use according to claim 2 or claim 3, wherein said treatment is treatment of MPM partially or fully resistant to a combination of chemotherapeutic agents comprising:
- cisplatin and pemetrexed; and/or
- cisplatin and raltitrexed.

5. The pharmaceutical composition for use according to claim 2, wherein said treatment is treatment of RCC partially or fully resistant to:
(a) therapeutic agents inhibiting mammalian/mechanistic target of rapamycin (mTOR); and/or therapeutic agents inhibiting vascular endothelial growth factor (VEGF) mediated formation of blood vessels, preferably via inhibition of tyrosine kinase activities and/or immunotherapeutic agents directly blocking VEGF; and/or
(b) immunotherapeutic agents promoting an immune reaction against said RCC.

6. The pharmaceutical composition for use according to claim 2 or claim 5, wherein said treatment is treatment of RCC partially or fully resistant to:
- mTOR inhibitors such as everolimus and/or temsirolimus; and/or tyrosine kinase inhibitors such as sunitinib and/or axitinib; and/or
- monoclonal antibodies such as avelumab, ipilimumab, nivolumab and/or bevacizumab.

7. The pharmaceutical composition for use according to claim 2, wherein said treatment is treatment of DLBCL partially or fully resistant to a combination of chemotherapeutic and immunotherapeutic agents such as a combination comprising several chemotherapeutic agents, a steroid and a targeted immunotherapeutic agent.

8. The pharmaceutical composition for use according to claim 2 or claim 7, wherein said treatment is treatment of DLBCL partially or fully resistant to a combination of chemotherapeutic and immunotherapeutic agents comprising rituximab, cyclophosphamide, doxorubicin hydrochloride, vincristine and prednisolone.

9. The pharmaceutical composition for use according to any one of claims 1 to 8, wherein said compound of Formula A is a compound of ***Formula A-1*** 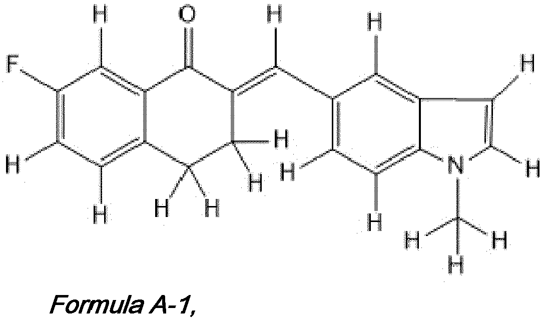 or a compound of ***Formula A-2***

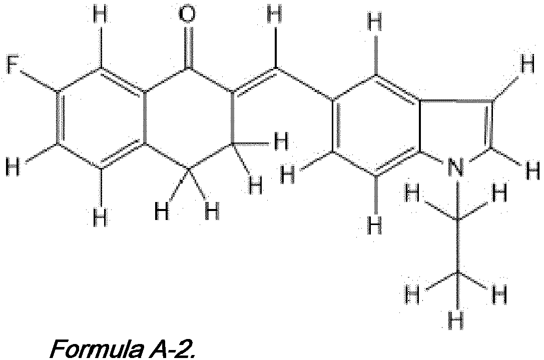

10. The pharmaceutical composition for use according to any one of claims 1 to 4 or 9, wherein said treatment is combination treatment of MPM also comprising the administration of platinum-based chemotherapeutic agents and/or folate antimetabolite chemotherapeutic agents.

11. The pharmaceutical composition for use according to any one of claims 1, 2, 5, 6 or 9, wherein said treatment is combination treatment of RCC also comprising the administration of chemotherapeutic agents inhibiting vascular endothelial growth factor mediated (VEGF-mediated) formation of blood vessels and/or immunotherapeutic agents promoting an immune reaction against said RCC.

12. The pharmaceutical composition for use according to any one of claims 1, 2, 7 to 9, wherein said treatment is combination treatment of DLBCL comprising the administration of several further chemotherapeutic agents and, optionally, a steroid and a targeted immunotherapeutic agent.

13. Use of a compound of ***Formula A*** 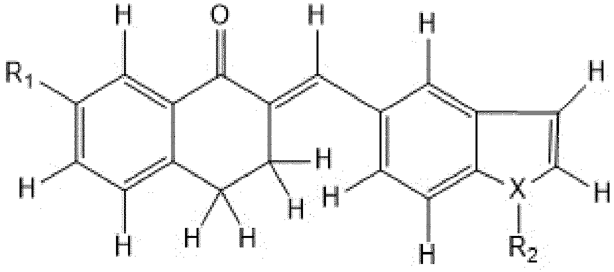 wherein:
X is a heteroatom;
R₁ is halo; and
R₂ is a C₁-C₁₀ alkyl,
for disturbing the polymerization of tubulin molecules.

14. Use of a compound of ***Formula A*** 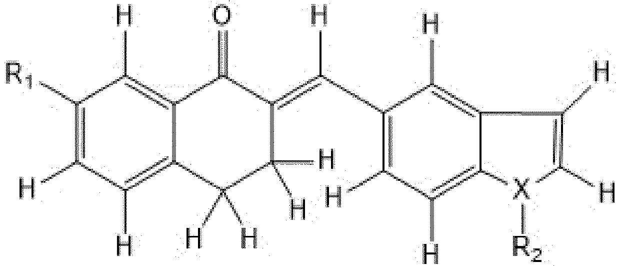 wherein:
X is a heteroatom;
R₁ is halo; and
R₂ is a C₁-C₁₀ alky,
for inhibiting formation of a functional spindle apparatus.

15. The use according to claim 13 or 14, wherein said compound of ***Formula A*** is a compound of ***Formula A-1*** 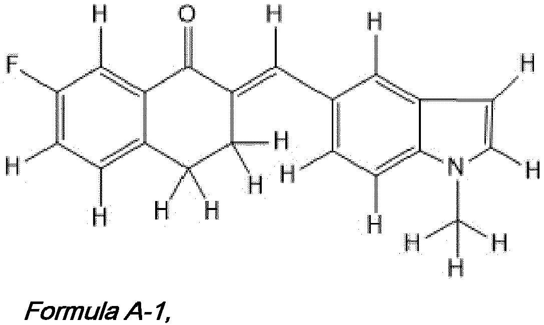 or a compound of ***Formula A-2***
